(19) Europäisches Patentamt
European Patent Office
Office européen des brevets

(11) EP 2 816 121 B1

(12) **EUROPEAN PATENT SPECIFICATION**

(45) Date of publication and mention of the grant of the patent:
**31.10.2018 Bulletin 2018/44**

(51) Int Cl.:
***C12Q 1/6886*** *(2018.01)*

(21) Application number: **14170193.8**

(22) Date of filing: **28.05.2014**

(54) **Method for obtaining information on hepatocellular carcinoma**

Verfahren zur Gewinnung von Informationen über hepatozelluläres Karzinom

Procédé permettant d’obtenir des informations sur le carcinome hépatocellulaire

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.05.2013 JP 2013113419**
**30.04.2014 JP 2014093759**

(43) Date of publication of application:
**24.12.2014 Bulletin 2014/52**

(73) Proprietors:
- **SYSMEX CORPORATION**
**Kobe-shi,**
**Hyogo 651-0073 (JP)**
- **The University of Tokyo**
**Bunkyo-ku,**
**Tokyo 113-8654 (JP)**

(72) Inventors:
- **Tai, Kaya**
**Kobe-shi**
**Hyogo 651-0073 (JP)**
- **Nagae, Genta**
**Tokyo 113-8654 (JP)**
- **Aburatani, Hiroyuki**
**Tokyo 113-8654 (JP)**

(74) Representative: **Paemen, Liesbet R.J. et al**
**De Clercq & Partners**
**Edgard Gevaertdreef 10 a**
**9830 Sint-Martens-Latem (BE)**

(56) References cited:
**WO-A2-01/26536**
**WO-A2-2008/008983**
**WO-A2-2012/167145**

- **MIN-AE SONG ET AL: "Elucidating the Landscape of Aberrant DNA Methylation in Hepatocellular Carcinoma", PLOS ONE, vol. 8, no. 2, 20 February 2013 (2013-02-20), page e55761, XP055173243, DOI: 10.1371/journal.pone.0055761**
- **JORDANA T. BELL ET AL: "Epigenome-Wide Scans Identify Differentially Methylated Regions for Age and Age-Related Phenotypes in a Healthy Ageing Population", PLOS GENETICS, vol. 8, no. 4, 19 April 2012 (2012-04-19), page e1002629, XP055151172, DOI: 10.1371/journal.pgen.1002629**
- **"Sample & Assay Technologies EpiTect Methyl II PCR Assay Handbook For regional DNA methylation analysis using MethylScreen(TM) technology", , 1 August 2012 (2012-08-01), XP055173408, Retrieved from the Internet: URL:http://www.qiagen.com/gb/resources/resourcedetail?id=0b366f6c-b6f7-4d3c-b82c-57a0fe1500e6&lang=en [retrieved on 2015-03-03] -& "EpiTect Methyl II PCR Assays - QIAGEN - EpiTect Methyl II PCR Primer Assay for Human CELF6 (CpG Island 104663)", , 3 March 2015 (2015-03-03), XP055173405, Retrieved from the Internet: URL:http://www.qiagen.com/gb/products/catalog/assay-technologies/epigenetics/epitect-methyl-ii-pcr-assays?catno=EPHS104663-1A#geneglobe [retrieved on 2015-03-03] -& Qiagen: "EpiTect Methyl II PCR Assays - QIAGEN - EpiTect Methyl II PCR Primer Assay for Human RNF135 (CpG Island 105884)", , 3 March 2015 (2015-03-03), XP055173410, Retrieved from the Internet: URL:http**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- KATE REVILL ET AL: "Genome-Wide Methylation Analysis and Epigenetic Unmasking Identify Tumor Suppressor Genes in Hepatocellular Carcinoma", GASTROENTEROLOGY, vol. 145, no. 6, 5 September 2013 (2013-09-05), pages 1424-1435.e25, XP055151069, ISSN: 0016-5085, DOI: 10.1053/j.gastro.2013.08.055

SEQUENCE LISTING

<110> SYSMEX CORPORATION THE UNIVERSITY OF TOKYO

<120> METHOD FOR OBTAINING INFORMATION ON HEPATOCELLULAR CARCINOMA AND MARKER AND KIT FOR OBTAINING INFORMATION ON HEPATOCELLULAR CARCINOMA

<130> SYSM-086-EP

<150> JP2013-113419
<151> 2013-05-29

<150> JP2014-093759
<151> 2014-04-30

<160> 10

<170> PatentIn version 3.5

<210> 1
<211> 4145
<212> DNA
<213> Homo sapiens

<400> 1

agttgaagag gtggcagtct gattccagct ctcagtcctt aggaatgtac ctttagctgt 60
tacccaaggc ccaccactcc tctcttagga agatcatgga ggatagttgg tgactctttc 120
ccagcaaaaa cttgaaagca cagcatggtg gtggtggtgg gggaggagat aagagtgttc 180
cttctcaaat catctcccct tctcctgccc atgtatgaca tgtgactctg gctctcttac 240
tccagccctg cctggacaga cttttctgca tgtaaacaag cctcctccct ctctctacag 300
gaagaaaggg gtgtggccga acaggaccgt gtatctctct agaaagggca gggagaagat 360
ctccttctgt ctcttcagcc tgactcaccc tgagaatggg cctggactgg gaccagcctg 420
tgaggggagg gaaagaaaga ggccataaag aggactgggg aggagcaaag aagaggcaca 480
ggagtgaggg gattagagaa ggaaagaagt gtggatttag ctgagagaaa tccaagaaga 540
aagggacaca gggtggcagg agttcccagc tgaggtcagg gttctgaact gaaatcaaaa 600
gaggtagagt gcattgggat tctggcgaag ggcaggagag attggggctg ttgttgaaca 660
tgagggggtg tctggaactg agagcctcct gggaattgaa ttggattttg tgttagacag 720
gtctgggttc tgaagaaggg gagggtttgg gtctgcggtg ttttaggttg gcagtctgag 780
agtcaaaaga aggctcttga gggggatggc cggactagga ggccagagga ggggtgcaag 840
tccaagatat gcatgtgggt ggttaggcct gagtgaggtg ccaccagatg gaggattcag 900
gagtgtttgg gctcagggtt tgggaaattg agggtattcg agatctagaa ggtggaataa 960
gttttcaaat tccagaattt taggtctgat gggagaggtg gtggaaaggt ccaattgaaa 1020
ggcagggact gctgggatgt gagaggtgga ggccaggaat ttggaggtta gagtggagaa 1080

-

catgttaggg gaccacagtg ataatcagag ggaggatgtc agagagaaaa ttctgtgatc 1140
tgggaagggg gctgggctga ggtggggctg atattggact ggtgttggac tggctctcgg 1200
cagggctagg gctggggctg ggctggggtc gggccacact ctaatcggat tggggctggg 1260
ctgggctggg ctgaccccgc gcacctttgt ggaggccggt gagccggtcc ttcagcaccg 1320
tcagctcgta gatgcggccg aactcctcga acagcggctt gaggtcctgc tcgtccaagc 1380
cccgcgggat ctgccccacg aagagcttga tggcgtcgtg gtccttcatg ggtacggcgg 1440
gaccggggtt tagcccgctc atgccgacgc cgctgtccgc ggtgctgaaa cccaggcgcg 1500
ggccggggcc agcgggctgc gctgaccctc ccggcgccgc ggccatgtcc ccgccctgtc 1560
agccctcccg ccggtcccac tggtcccgcc tgtcccgccg tcccctccct ggaccggtgg 1620
cgagggccag gggaggggg cggagcccgg gcggagaggg cgggggctg cccaggggc 1680
ggggtccggg tggaggggcg tagaggggt ggggcgggca ggaaagggc ggggccgggg 1740
cggggcgggc ccgggccgag gtggcggctg aacgctgggg tctggcttga ggtccccgtg 1800
cggctctctc tgggctcccg cccgagctct cccagagccg agccccgagc cccagccccc 1860
gtgcttggtg acgtcaggca gctggccgcc gagtctacgc aaatgatttg cataatgagg 1920
aagcagccgc caatcagagt gggagttgac tgggctcgcc gggggcgggg ggggcggctg 1980
ggctcacgcg ctcctgctcg ccatggcaac cggaccctcg aatgccctgt gtgtgtcgag 2040
tgtgtgcggg cgtggccgtg gatcgcgcgt gtgcgccttg ggccgcgtga ggggaagcag 2100
cttcgtcatg cgtgatggtg tctgccggag tcggggctaa tccccaccgc acacacaggc 2160
acacacaggc aaacgcaggc acacgcaggc acacacacct ccaaacgctg gtgcgtttcc 2220
tgcgggggtg gatggggatt cctcttctct attcccacta tcgagccagt ccctcggagc 2280
caatcccacc ccgccccatc acacatctct gtcttcggtg tctttaacca ctttcttacc 2340
cctgagcctc agacacattc gtctcccttg cagccttaaa aagaaaggaa gaataaaata 2400
aaaacccctt aaacactcct tgaacacgga ttcctcctta ggagagtgag ggaacttagc 2460
cgtatttact cagaacccat tatgcaccag gtgcccagtt catcccagtt ctgtgaactc 2520
aggagtgttc tctttttccg gaaaaggaag ctgaggttct gagggtcgag gatgttaggg 2580
ccaggcccac cccaacattt gttgggccca gcgcaaaata agaaatggaa atcgacatac 2640
tgtgcatcta aacatttaaa agatacaaat caaattacaa tttgttaaat atgttcccct 2700
cttattttca caaatacagg ttcagaatta cgaaaatgaa agacatatgt acagctacgc 2760
tttctatatg actgaaagtt ggcaaaatat cagagctgat ggaattttat tactcctgca 2820
catgtctagg tattctcttg atggatcagt gatgtttgca caaagtagta atacaatgtt 2880
tggacaaaga cacatacttc ataaattata tatttattcc ataatatatt tttcatgcct 2940
ctttcagtaa aattgctaat tattttaata gagatttcct tttttttttt ttgataggtt 3000

```
atcattctgt cacccaggct gtagtgcagt ggtgcgatct aagatcactg cagcctcaac   3060
ctcctgggtt caagcgatcc tcccacctaa gcctcccaag tagctgggat gacaggtgca   3120
cgctaccatg cctggataat tttttttctt tttttttttt tgtagagatg gtggtttcac   3180
cattctgccc aggcttgtct agaacactcc tgagctcaag tgatccaccc acctcggtgc   3240
cccagagtgc tgggattaca ggagtgtgcc actgcacctg gcctaataga gattttaaca   3300
taacttgtgt ccattgacta ttctaagaaa ctgctggaga ccagcagtag caactggaat   3360
catcaaaaag ttcttaaaac aatattcaat tcagaagcaa atcatgattt ttaaatgatt   3420
tatttttctg gctgagcatg gtgactcaca cctgtaatcc cagcactttg ggaggccaag   3480
gcgggcagat cacttgaggt caggagttca agaccagcct ggccaacatg acaatatccc   3540
gtctctacta aaaatacaaa aattagccag gcatggtggc gggctcctcc tactctggag   3600
gctgaagcag gagaatcgtt tgaacccacg aggtggaggt tgcaatgagc caagatcgtg   3660
tcactgcact ccagcctggg tgacagagtg agattccatc tcaaaaaaaa aaaaaaaatg   3720
cagcagcaaa ttatgatttt caaaaatcat ttttctggcg ggatgcggtg gctcacacca   3780
gtaatcccag cacttcagga ggccgaggtg ggtggattgc ttgagatcag gagtttgaga   3840
ccagcctggc caatatggca aaaccccatt tttactaaat gaataaataa ataaaataaa   3900
ttatttttct cctttaacag aaagttactc cttgtaataa gttgaccacg aaatgggtta   3960
ctggaatttc attttcattc agtcctatgt agtttttttt tttttttttt ttttgagatg   4020
gagtcttgct ctgtcgccca ggttggagtg cactggcccg atctcagctc actgcaagct   4080
ccgcttcccg ggttcacgcc attctcctgc ctcagcctcc cgagtagctg ggactacggt   4140
tgccc                                                                4145
```

<210> 2
<211> 4095
<212> DNA
<213> Homo sapiens

<400> 2

```
tcaggcaggt ctcgaactcc tgagttcagg caatccacct gcctcagcct cccaaagtgc      60
tgagattaca ggcataagcc accacgcccc gccagtgatt tcatttttta agttggaaga     120
tctgagcatg tttaagtgtt tagtatcatg cccgttttac agaaaaaaag aggctcttag     180
aaactacgat tttactccga tgcggagatt ctagaacaag atccaggtaa tacatcagtc     240
aggataggct aggttatgct gtagcagcac acacccacta aatctcagta agctgaatac     300
atcctagttt tcttctccct cttactacgt atcctagggt gcgttggcag ggagtctgct     360
catcttagcc actcagtgac ccaggtcacc ggatgatggt ttcatctcca taattggggc     420
agctggcaga aagggataca atggccggct cagtcgcctt taaagctggt gggaagtaac     480
```

atattacctc tgtgtgtttc aagtgggtca gggaagtgcc agtcctacca tgtgcccaga 540
aagggaaagc actggaaacg ttgatgagca gcgctgccta ctccatgaca cttccagtat 600
tagaaataac cacaaacgct tttttttctt tcttttcttt cttttttttc tttttttttt 660
tttttttttt tttgagacgg agtctcccta tgtcgcccag gctggagtgc agtggcgcag 720
tcttggctca ctgaaacctc cgcctcccgg gttcaagaga ttctcctgcc tcagcctccc 780
aagtagctgg gattacaggc atgcgccagg gcgcctggct tttcttttct tttctttttg 840
tattttcagt agagatgagg gttcaccatg ttggccaggc tggtcttgaa ctcctgactt 900
caagtgatcc aaaggtcggc tacaaacttg tattatctga tacctacttt gagcccaggc 960
cagaaaggat aaagtaaagc atgagacgtt gttagttcat ttcaacagat tggggagttg 1020
tgggttttag tctctgacgc ttcattgtta ccatgagagc ctgcaagttt ttcttttttc 1080
tctttttttt ttttttaacc tctttgggtc atctgggagg acgtggtgct cttttagctc 1140
taacattttc tggttgcaat gcgtttagga ctgggatgcg aagtgagtga gtgagtgagc 1200
cttctcagga aaacactttc tagaattttg tccgagtgtc tgttgggaaa cgcgcttaca 1260
tcccgcagaa ggcggtaagg acacagcgca caactctgca ttatacctgc gagttaaaaa 1320
gccaagggtt cgctcactca accttgaatg cgcgtcagaa tcacctgggg gaggttttac 1380
aaaatacccg tgctccaccc aagacaatga aatcagactc cctgcgagcg gggccgaaca 1440
tcagcatttt tgaggacgct ccctgtggct gtgcatcggt gatctttaat cccctgctac 1500
ttgttagagg agctggatga cacttcgtgg gctcattctc ataaataagc tgctgaatgg 1560
agtcctctcc tcccaagagt tgtcggtgtt gcatcttttt tgttgtttat tttttttttaa 1620
gccaacggac gaagacatcc aacggtgctg catcttaatg gagggacatc cagctaaagg 1680
gaatcttgga gacctccgcg ggtttctagc agctcgcggc atgttggttt cccagggcaa 1740
gaggccgcca ctgaaggtca gctctgtctt tccagctggg cactggaggc tctaagtctg 1800
ttttcagcag gatcggagga aggagacggg gtggcgccaa ggaaggagga gaaaaggcgg 1860
ccgagaaaag gaggagggca aggggaagag gaagggcgag ggaggagcct gaggagactc 1920
gcccggctca accccgacgt ccgcgccccg gccgcctgtt ggccatggcg ggcctgggcc 1980
tgggctccgc cgttcccgtg tggctggccg aggacgacct cggctgcatc atctgccagg 2040
ggctgctgga ctggcccgcc acgctgccct gcggccacag cttctgccgc cactgcctgg 2100
aggccctgtg gggcgcccgc gacgcccgcc gctgggcctg ccccacttgc cgccagggcg 2160
ccgcgcagca gccgcacctg cggaagaaca cgctactgca ggacctggcc gacaagtacc 2220
gccgcgccgc acgcgagata caggcgggct ccgaccctgc ccactgcccc tgcccgggct 2280
ccagttccct ctccagcgcg gccgcgaggc cccggcgccg cccggaactg cagcgggtag 2340

ggaggccggg cccgcagctc ccctggctcc cccgggctgc ccgccgcctg accctttccc 2400
atgtggctcg aacccctttc ctcagccgtt ctacttttac gttccttttc tcagtctaaa 2460
agtcgagttc cgctcttcgg aggcactttg gaaagttcat aaaagtatga agaagtagaa 2520
aaaaacaaat tccccatctt ccgaaagctt gtcaacttag ctgttacaca gcttggcata 2580
tttcaacttc ttcccaatcg atcttcggtc tctttctctg aaacttgtaa aattgtggta 2640
agatctatat aacattaaaa ctggccattt taaccttttt ctttatttct ttgagacgga 2700
gtctcgctct gtcgcccagg ctggatggag tgcaatggcg tgcgtgatct cggctcactg 2760
caacctccgc cccccagggt cgagtgattc tcttgcttca ggctcgagag tagctgggat 2820
tacaggcgtg cgccatcacg cccggctaat tttggtattt ttagtagaga cggggtttcg 2880
acatgttagc caggctagtc tcaaactctt gaccacagat gatccgtccg cctcggcctc 2940
ccaaagtgct gggattacag tcatgagcca cagcgcctgg cctcatttta accttttatt 3000
tttttgagac ggagttttgc tttgtggccc aggctggagt gcagtggcgc catcttgtct 3060
cactgcaagc tccgcctccc gggttcacgc cattctcctg cctcagcctc ccgagtaact 3120
gggactacag gcgcccgcca ccacgcctgg ctaatttttt gtatgttttt ttcttttttt 3180
aaaaaatagg gacggggttt caccgttgtt agccaggatg gtctcgatct tctgacctcg 3240
tgatctgccc gcctcggctt cctaaagtgc tgggattaca ggtgtgagcc accgcgcccc 3300
gcctatttta accattttta agtgtacaat tcagtgacaa attagttaca tttcctgttg 3360
tgcaactatc acttctgttt ccaaaactgt ttcatcatca taaacagaaa ctttgtactc 3420
attaagcagt aactcctcat ttctcctccc tctagcccct gctcacatct aagctacttt 3480
ctgtatctga gtttgcctgt tctagatatt tcatataact ggaatcgttc aatgtttgtc 3540
cctttgtgcc tggtttcttt cacttagcat agtgttttca aagttcattc atgttgtagc 3600
atgtgtcaga actttattcc ttttatgggg ctgaatgata ttccattggg tggatgtgcc 3660
acattttttt tgtttgtttt gttttgagat ggagtcttgc tctgttgcca ggctggagtg 3720
tagtggcatg atcttggctc actgcaacct ctgcccccctg ggttcaagtg attctcctgc 3780
ctcagcctcc caagtagctg ggactacagg catgcaccac cacacccagg taatttttgt 3840
atttttagta gagacgggtt ttcaccatgt tggccaggat ggtctcgatc tcttgacctt 3900
gtgatctgcc cacctcggcc tccggaagtg ttgggattac aggcgtgagc caccgcaccc 3960
agcctcactg tgattttgat ctggatttcc ttaatgacta atgatgttgc ccatcttttc 4020
ttgtgcttgt tggtcattta tagatcttca ttgcagaaat gtctattcaa gtcctttgac 4080
cattttaaaa ttggg 4095

<210> 3

<211> 23
<212> DNA
<213> Artificial Sequence

<220>
<223> primer

<400> 3
ttttcgaatg ttttgtgtgt gtc 23

<210> 4
<211> 19
<212> DNA
<213> Artificial Sequence

<220>
<223> primer

<400> 4
aaaaattaac cccgactcc 19

<210> 5
<211> 19
<212> DNA
<213> Artificial Sequence

<220>
<223> primer

<400> 5
gttggtcgag gacgatttc 19

<210> 6
<211> 25
<212> DNA
<213> Artificial Sequence

<220>
<223> primer

<400> 6
aacaataacg acaaaaacta taacc 25

<210> 7
<211> 33
<212> DNA
<213> Artificial Sequence

<220>
<223> primer

<400> 7
gggatattaa gtggagttat tttggtttta gtt 33

<210> 8
<211> 21
<212> DNA
<213> Artificial Sequence

<220>
<223> primer

<400> 8
ccctcccaac atccttccta a 21

<210> 9
<211> 145
<212> DNA
<213> Artificial Sequence

<220>
<223> bisulfite-treated DNA

<400> 9

```
uuatgguaau cggauuutcg aatguuutgt gtgtgtcgag tgtgtgcggg cgtggucgtg       60

gatcgcgcgt gtgcguuttg ggucgcgtga ggggaaguag uttcgtuatg cgtgatggtg      120

tutgucggag tcggggutaa tuuuu                                            145
```

<210> 10
<211> 95
<212> DNA
<213> Artificial Sequence

<220>
<223> bisulfite-treated DNA

<400> 10

```
tggutggucg aggacgauut cggutguatu atutguuagg ggutgutgga utgguucguu       60

acgutguuut gcgguuauag uttutgucgu uautg                                  95
```

## Description

### TECHNICAL FIELD

The present invention relates to a method for obtaining information on hepatocellular carcinoma in a subject. Also described herein is a marker and a kit used in the method.

### BACKGROUND ART

Hepatocellular carcinoma is a type of primary liver cancer and is a malignant tumor originated from hepatocytes. Hepatocellular carcinoma is almost inactive in the initial stage. However, when subjective symptoms such as abdominal lumps, sudden abdominal pain or jaundice are manifested, the disease has already developed to severe stages in most cases. Hepatocellular carcinoma is known to often develop in patients with chronic hepatitis resulting from hepatitis viruses or with hepatic cirrhosis. Thus early detection of hepatocellular carcinoma has been sought by identifying the patients as a high-risk group and proactively examining the patients.

Examination for hepatocellular carcinoma includes measurement of tumor markers in blood. The tumor markers currently used include AFP (a-fetoprotein), PIVKA-II (protein induced by vitamin-K absence II) and the like. The examination of the tumor markers is used to screen patients with hepatocellular carcinoma; however the markers have insufficient sensitivity and specificity. For example, the examination of AFP may not result in an abnormal value for a hepatocellular carcinoma with a relatively small size and the examination of PIVKA-II may provide false positives for subjects with vitamin K deficiency or subjects taking warfarin. Therefore definitive diagnosis of hepatocellular carcinoma is made, in addition to the examination of tumor markers, by ultrasonic examination and examination by imaging such as CT and MRI.

Meanwhile, new diagnosis methods for cancer have been recently studied which are based on genetic information. The methods include, for example, ones based on information on methylation of DNAs. The methods use markers which are CpG sites (5'-(CG)-3') in base sequences of certain genes. Based on the analysis results of the methylation status of the markers, information such as the presence or absence of a cancer cell is obtained, which information may be used as an index for diagnosis of cancer.

Methods for determining cancer utilizing methylation analyses of DNA have been studied and developed for hepatocellular carcinoma. For example, the publication by Lee S. et al. (Am J Pathol; 163(4): 1371-78)) discloses that genes such as GSTP1, p16 (also referred to as CDKN2A) and E-cadherin (also referred to as CDH1) are not methylated in non-cancerous liver tissues while the genes are highly methylated in tissues of hepatocellular carcinoma.

Song M-A et al. (Plos One; 8(2):e55761) describe genome-wide methylation profiling of hepatocellular carcinoma.

### SUMMARY OF INVENTION

Technical Problem

Although some genes indicative of abnormal methylation in hepatocellular carcinoma have been reported as described above, these genes include genes which are methylated to some extent in a type of cancer different from hepatocellular carcinoma. When methylation analysis is carried out with a marker that is methylated in several types of cancer including hepatocellular carcinoma, information deduced from the analysis result may include not only information on hepatocellular carcinoma but also information on other types of cancer. In this case, even when an analysis result provides information indicating that a sample contains a cancer cell for example, it is difficult to determine whether the cancer cell is derived from hepatocellular carcinoma or from other another type of cancer.

For this reason when obtaining information on hepatocellular carcinoma by methylation analysis of a marker, the specificity of the marker to hepatocellular carcinoma is very important. Thus there is a need for a novel marker specific to hepatocellular carcinoma, which allows specific detection of a cancer cell derived from hepatocellular carcinoma.

Accordingly an object of the present invention is to provide a method for obtaining information on a cancer cell derived from hepatocellular carcinoma by identifying such a novel marker and analyzing methylation status of the marker.

Solution to Problem

The present inventors have identified novel markers which are genetic regions specifically methylated in DNAs obtained from cancerous tissues of hepatocellular carcinoma. The present inventors have found that cancer cells derived from hepatocellular carcinoma can be clearly discriminated from other cells (cells of normal tissues, cells of non-cancerous tissues and cancer cells derived from a type of cancer other than hepatocellular carcinoma) based on the result obtained by analyzing the methylation status of the markers, thereby achieving the present invention.

Thus the present invention provides a method for obtaining information on the presence or absence of a cancer cell derived from hepatocellular carcinoma in the biological sample collected from the subject comprising the steps of:

preparing a DNA sample from a biological sample collected from a subject;
analyzing methylation status of a promoter region of at least one marker gene specific to hepatocellular carcinoma, the marker gene being selected from CELF6 (CUGBP, Elav-like family member 6) and RNF135 (Ring finger protein 135) in the DNA sample obtained from the preparation step;
wherein the analyzing step is the step of analyzing the presence or absence of methylation of at least one CpG site in the promoter region corresponding to a region consisting of SEQ ID NO: 9 or 10 wherein U is replaced with C; or analyzing methylation frequency; and
obtaining information on hepatocellular carcinoma in the subject based on an analysis result obtained from the analyzing step,
wherein the biological sample contains a cancer cell derived from hepatocellular carcinoma when the analysis result shows that there is a methylated CpG site, or when the methylation frequency obtained from the analyzing step is higher than a predetermined threshold.

Also described herein is a marker for obtaining information on hepatocellular carcinoma by methylation analysis, which is at least one CpG site selected from CpG sites located in promoter regions of the CELF6 and RNF135 genes.

Also described herein is a kit for obtaining information on hepatocellular carcinoma, comprising a primer set for analysis of methylation status of at least one CpG site selected from CpG sites located in promoter regions of the CELF6 and RNF135 genes.

Also described herein is a marker for obtaining information on hepatocellular carcinoma, which is a polynucleotide obtained by subjecting an isolated DNA to bisulfite treatment, wherein the isolated DNA consists of a base sequence corresponding to the whole or a partial promoter region of the CELF6 or RNF135 gene and contains at least one CpG site in the promoter region and at least one cytosine not included in CpG sites.

Advantageous Effects of Invention

The present invention can provide a novel marker which allows provision of information on hepatocellular carcinoma. The present invention can also provide a method for obtaining information on hepatocellular carcinoma in a subject by analyzing the methylation status of the marker.

## BRIEF DESCRIPTION OF DRAWINGS

FIG. 1(A) is a graph showing the methylation positive rate of the promoter region of CELF6 gene in DNA extracted from normal liver tissues, and cancerous tissues and non-cancerous tissues of hepatocellular carcinoma;
FIG. 1(B) is a graph showing the methylation positive rate of the promoter region of RNF135 gene in DNA extracted from normal liver tissues, and cancerous tissues and non-cancerous tissues of hepatocellular carcinoma;
FIG. 2(A) is a graph showing the methylation positive rate of the promoter region of CELF6 gene calculated from methylation data of various clinical specimens;
FIG. 2(B) is a graph showing the methylation positive rate of the promoter region of RNF135 gene calculated from methylation data of various clinical specimens;
FIG. 3(A) is a graph showing the methylation positive rate of the promoter region of a known marker gene CDH1 calculated from methylation data of various clinical specimens;
FIG. 3(B) is a graph showing the methylation positive rate of the promoter region of a known marker gene CDKN2A calculated from methylation data of various clinical specimens;
FIG. 3(C) is a graph showing the methylation positive rate of the promoter region of a known marker gene GSTP1 calculated from methylation data of various clinical specimens;
FIG. 4 is an image showing the results of methylation-specific PCR (MSP) amplification of DNA extracted from normal liver tissues, and cancerous tissues and non-cancerous tissues of hepatocellular carcinoma using the respective primer sets for CELF6 and RNF135;
FIG. 5 is an image showing the results of MSP amplification of DNA extracted from plasma from healthy subjects and plasma from hepatocellular carcinoma patients using the respective primer sets for CELF6 and RNF135;
FIG. 6 is an image showing the results of MSP amplification of DNA extracted from cancer tissues other than hepatocellular carcinoma and normal tissues using the respective primer sets for CELF6 and RNF135 and the primer set for accuracy control;

FIG. 7(A) is a graph showing the band intensity of amplification products obtained by MSP amplification of DNA extracted from cancer tissues other than hepatocellular carcinoma and normal tissues using the primer set for CELF6;

FIG. 7(B) is a graph showing the band intensity of amplification products obtained by MSP amplification of DNA extracted from cancer tissues other than hepatocellular carcinoma and normal tissues using the primer set for RNF135;

FIG. 7(C) is a graph showing the band intensity of amplification products obtained by MSP amplification of DNA extracted from cancer tissues other than hepatocellular carcinoma and normal tissues using the primer set for accuracy control;

FIG. 8 is a schematic view of an example of a determination device for providing information on hepatocellular carcinoma in a subject;

FIG. 9 is a block diagram showing the functionality configuration of the determination device of FIG. 8;

FIG. 10 is a block diagram showing the hardware configuration of the determination device in FIG. 8; and

FIG. 11 is a flow chart of determination for providing information on hepatocellular carcinoma in a subject using the determination device in FIG. 8.

## DESCRIPTION OF EMBODIMENTS

In the method for obtaining information on hepatocellular carcinoma of the present invention (hereinafter also merely referred to as "method"), a DNA sample is first prepared from a biological sample collected from a subject.

In the present embodiments, the biological sample is not particularly limited as far as it is a biological sample containing DNA of a subject and is preferably a sample containing a genomic DNA such as a clinical specimen. The clinical specimen may include, for example, body fluid, urine, tissues obtained by operations or biopsies and the like. The body fluid may include blood, serum, plasma, lymph fluid, ascetic fluid, bone marrow aspirate, nipple discharge and the like. The biological sample may also be a culture obtained by culturing cells or tissues collected from a subject.

The DNA sample can be prepared by extracting DNA from the biological sample. The methods for extracting DNA from biological samples are well known in the art. Extraction of DNA can be carried out, for example, by mixing the biological sample with a treatment solution containing a surfactant for solubilization of cells or tissues (e.g. sodium cholate, sodium dodecyl sulfate etc.), and subjecting the resulting mixture to a physical procedure (stirring, homogenization, ultrasonication etc.) to release DNA contained in the biological sample into the mixture. In this case, it is preferable to centrifuge the mixture to precipitate cell debris and use the supernatant containing the released DNA in the next step of analyzing. The obtained supernatant may be purified according to well-known methods. DNA can also be extracted and purified from a biological sample by using commercially available kits.

The preparation step preferably further comprises the step of fragmenting the extracted DNA. By fragmenting DNA to have appropriate length, methylated DNA immunoprecipitation (MeDIP) and non-methylated cytosine conversion as described hereinbelow can be effectively carried out.

Fragmentation of DNA may be carried out by ultrasonication, alkaline treatment, restriction enzyme treatment and the like. When DNA is fragmented by alkaline treatment, a sodium hydroxide solution may be added to a DNA solution to the final concentration of 0.1N to 1.0N and the mixture may be incubated at 10°C to 40°C for 5 to 15 minutes to fragment the DNA. In case of the restriction enzyme treatment, the restriction enzyme may appropriately be selected based on the base sequence of DNA, which may be *Mse*I or *Bam*HI, for example.

According to the present method, the methylation status of a CpG site in a promoter region of at least one gene selected from CELF6 and RNF135 in DNA obtained from the preparation step is analyzed.

As used herein, the term "CpG site" means a site of a sequence in which cytosine (C) and guanine (G) are adjacent in this order from 5' to 3'. The letter "p" in "CpG" represents a phosphodiester bond between cytosine and guanine.

As used herein, to "analyze the methylation status" means to analyze the presence or absence of methylation of a CpG site located in a promoter region of at least one gene selected from CELF6 and RNF135 corresponding to a region consisting of SEQ ID NO: 9 or 10 wherein U is replaced with C; or analyze methylation frequency in the promoter region.

The base sequences *per se* of the promoter regions of CELF6 and RNF135 genes are well known in the art. The base sequences can be obtained from well-known databases such as the one provided by the National Center for Biotechnology Information (NCBI) (http://www.ncbi.nlm.nih.gov/). The ID numbers of the above genes are shown in Table 1. The base sequences of the promoter regions of the genes are represented by SEQ ID NOs: 1 and 2, respectively.

Table 1

| Gene symbol | Unigene ID | Entrez ID | Transcript ID | SEQ ID NO: |
|---|---|---|---|---|
| *CELF6* | Hs.348342 | 60677 | NM_052840<br>NM_001172684<br>NM_001172685 | 1 |
| *RNF135* | Hs.29874 | 84282 | NG_011701<br>NM_032322<br>NM_197939 | 2 |

In particular embodiments of the present invention, the analyzing step may be the step of analyzing the presence or absence of methylation of at least one CpG site among CpG sites located in a promoter region corresponding to a region consisting of SEQ ID NO: 9 or 10 wherein U is replaced with C of at least one gene selected from CELF6 and RNF135. The term "presence or absence of methylation" means whether or not cytosine in a CpG site located in the promoter region is methylated. In a particular embodiment, one CpG site may be analyzed; however, more than one CpG site is preferably analyzed for the presence or absence of methylation. More than one CpG site may be selected from a promoter region of one gene or from each of promoter regions of more than one gene.

In a particular embodiment of the present invention, the analyzing step may be the step of analyzing the methylation frequency in a promoter region of at least one gene selected from CELF6 and RNF135. The term "methylation frequency" means a ratio of the number of methylated CpG site(s) relative to the number of CpG site(s) located in the promoter region. Also described herein, the target for analysis may be the whole promoter region or a part thereof including at least one CpG site. The target for analysis may contain only one CpG site; however it is preferable that the target for analysis contains more than one CpG site. The target for analysis may be selected from any one promoter region among the above genes or from promoter regions of more than one gene.

The positions and numbers of CpG sites located in the promoter regions of CELF6 and RNF135 genes are already known. Thus the number of methylated CpG site(s) itself in the promoter regions can also be used as the methylation frequency.

The methylation frequency may be a "methylation score" obtained by analyzing DNA with mass spectrometry such as MassARRAY® as described hereinbelow. MassARRAY® allows calculation of methylation score based on the ratio between the area of a peak derived from a methylated DNA fragment and the area of a peak derived from a non-methylated DNA fragment obtained after measurement of the DNA fragments.

In particular embodiments of the present invention, the target for analysis may be any CpG site(s) or region(s) including the CpG site(s) in the promoter regions corresponding to a region consisting of SEQ ID NO: 9 or 10 wherein U is replaced with C of CELF6 and RNF135 genes without particular limitation. The positions and numbers of CpG sites located in the promoter regions of the genes are already known. Thus the target CpG site or region may be appropriately selected by a person skilled in the art based on the routine experiments according to the well known analysis methods described hereinbelow.

Various methods are well-known in the art as to the method for analyzing methylation status. According to the present method, it is not specifically limited as to which analysis method is used; however, the analysis method preferably comprises differentiating methylated DNA from non-methylated DNA, amplifying DNA and detecting methylated DNA and/or non-methylated DNA.

The step of differentiating methylated DNA from non-methylated DNA may include the step of carrying out methylation sensitive restriction enzyme treatment, a MeDIP method, non-methylated cytosine converting treatment and the like.

The step of amplifying DNA may include the step of carrying out PCR, quantitative PCR, IVT (*in vitro* transcription) amplification, SPIA™ amplification and the like methods.

The step of detecting methylated DNA and/or non-methylated DNA may include the step of carrying out electrophoresis, sequence analysis, microarray analysis, mass spectrometry, Southern hybridization and the like.

The MeDIP method is a method in which methylated DNA in a biological sample is concentrated by immunoprecipitation using an anti-methylated cytosine antibody or an anti-methylated cytidine antibody, or an antibody which specifically recognizes a methylated DNA-binding protein. In embodiments of the present invention, the analyzing step may be the step of concentrating methylated DNA in DNA obtained in the extracting step by the MeDIP method and analyzing the methylation status of the concentrated methylated DNA. The methylated DNA concentrated by the MeDIP method may be amplified by e.g. IVT amplification and the methylation status of the obtained amplified product may be analyzed by using a microarray. These analysis procedures are referred to as the MeDIP on chip method.

The non-methylated cytosine converting treatment is the one in which DNA extracted from a biological sample is subjected to reaction with a non-methylated cytosine conversion agent so as to convert non-methylated cytosine(s) in the DNA to a different base (uracil, thymine, adenine or guanine). In this context, the non-methylated cytosine conversion agent is a substance which can react with DNA and convert a non-methylated cytosine in the DNA to a different base (uracil, thymine, adenine or guanine). The non-methylated cytosine conversion agent suitably used may be, for example, bisulfite such as sodium, potassium, calcium or magnesium bisulfite.

In the treatment using bisulfite, non-methylated cytosine(s) in DNA is converted to uracil due to deamination reaction, while a methylated cytosine does not undergo such a base conversion.

Thus, the difference in methylation status of a CpG site in DNA is converted to the difference in a base sequence (C and U) by the non-methylated cytosine converting treatment using bisulfite. The non-methylated cytosine converting treatment using bisulfite is referred to as bisulfite treatment.

When the bisulfite treatment is carried out, the amount (concentration) of bisulfite added is not specifically limited as long as it can sufficiently convert non-methylated cytosine(s) in DNA, and corresponds to, for example, 1M or higher, preferably 1M to 15M, more preferably 3M to 10M as the final concentration in a solution containing DNA. The incubation conditions (temperature and time) after addition of bisulfite may be appropriately selected according to the amount added of bisulfite, and for example, when bisulfite is added at a final concentration of 6M, the incubation is carried out at 50°C to 80°C for 10 minutes to 90 minutes.

Methylation status of CpG sites in DNA can be analyzed by analyzing the sequence of DNA after bisulfite treatment and detecting the difference in base sequence from the original sequence. This process is referred to as a bisulfite sequencing method.

Methylation status of CpG sites can be alternatively analyzed by mass spectrometry. Specifically, a DNA after bisulfite treatment as a template is amplified by PCR using a primer set specific for a base sequence which is a target for analysis, and the obtained PCR product is subjected to IVT amplification to convert methylated cytosine and uracil respectively to guanine (G) and adenine (A). The obtained IVT amplification product is digested with RNase A and the difference in mass (16 Da) due to difference between G and A between the obtained digested fragments is detected on a MALDI-TOF (matrix assisted laser desorption/ionization time-of-flight) mass spectrometer to analyze methylation status of DNA. This method is referred to as MassARRAY® analysis.

It is known that the cleavage site in IVT products by RNase A is between an arbitrary base and the adjacent uracil (U) or thymine (T). Thus the base sequence and mass of the IVT product cleaved by RNase A may be predicted based on the base sequence of the template DNA. Accordingly the peaks obtained in MassARRAY® can be identified as to the portions of the base sequences in the template DNA from which the peaks originate. For example, when one CpG site is methylated in a DNA fragment, a peak obtained in MassARRAY® shifts to the side with an increased mass for 16 Da. When a DNA fragment containing more than one CpG site is analyzed for example, the DNA fragment having two methylated CpG sites shows a shift of 32 Da and the DNA fragment having three methylated CpG sites shows a shift of 48 Da.

In mass spectrometry such as MassARRAY®, the methylation score of the analyzed DNA fragment can be calculated. For example when a DNA fragment having a certain sequence results in the ratio between the area of the peak of the non-methylated DNA fragment and the area of the peak of the methylated DNA fragment obtained in a resulting chart from the analysis of 1:3, the methylation score of the DNA fragment is 0.75 (= 3/(1+3)). The methylation score is theoretically 1 for a DNA fragment in which all CpG sites are methylated and 0 for a DNA fragment without any methylated CpG site.

Methylation status of CpG sites can be analyzed by a methylation-specific PCR (MSP) method. The MSP method is a method in which methylation status of CpG sites (presence or absence of methylation) is analyzed by amplifying DNA after bisulfite treatment by PCR using a primer set described hereinafter and determining presence or absence of a PCR product.

The MSP method utilizes a primer set which can amplify a base sequence having a CpG site to be analyzed that is methylated (i.e. cytosine is not converted to uracil) but cannot amplify a base sequence having a CpG site that is not methylated (i.e. cytosine is converted to uracil). According to the MSP method using such a primer set, presence of a PCR product indicates methylation of the CpG site analyzed.

The MSP method may also be carried out by using a primer set which cannot amplify a base sequence having cytosine in a CpG site to be analyzed that is not converted to uracil but can amplify a base sequence having cytosine in a CpG site that is converted to uracil. In this case, absence of a PCR product indicates methylation of the CpG site analyzed.

Each primer in the primer set used for the MSP method may be appropriately designed by a person skilled in the art based on the base sequence including a CpG site to be analyzed, and it is preferably designed so as to contain cytosine of the CpG site to be analyzed at the 3' end or in the vicinity thereof of the primer.

Methylation status of CpG sites may alternatively be analyzed with a microarray. In this case, the microarray for analysis may be prepared by immobilizing a nucleic probe complementary to the base sequence of a promoter region of CELF6 or RNF135 gene on a substrate. The microarray can be prepared according to well-known methods in the art.

In the analysis using a microarray, DNA extracted from a biological sample is preferably labeled with a labeling substance well-known in the art. Thus, the present method preferably further comprises the step of labeling the extracted DNA. The labeling step is advantageously carried out after the DNA amplifying step because all DNA in the biological sample may be labeled. The labeling substance may include fluorescence substances, haptens such as biotin, radioactive substances and the like. The fluorescence substances may include Cy3, Cy5, FITC, Alexa Fluor™ and the like. Labeling of DNA facilitates measurement of a signal from a probe on the microarray. A method for labeling DNA with the labeling substance is well-known in the art.

The above signal may be any suitable signal depending on the type of microarray. For example, the signal may be an electric signal generated when a DNA fragment hybridizes to a probe on the microarray, or a fluorescence or luminescence signal generated from a labeling substance when DNA to be analyzed is labeled as described above. Detection of a signal can be carried out by using a scanner comprised in a conventional microarray analyzer. The scanner may be, for example, GeneChip® Scanner3000 7G (Affymetrix), Illumina® BeadArray Reader (Illumina) and the like.

In the present method, information on hepatocellular carcinoma in the subject is obtained based on the analysis result obtained in the analyzing step. The information on hepatocellular carcinoma is not particularly limited as long as it may be an index on diagnosis of hepatocellular carcinoma and is preferably information indicative of the occurrence or status of hepatocellular carcinoma or both thereof in a subject. The information may include, for example, the presence or absence of a cancer cell derived from hepatocellular carcinoma in a biological sample collected from a subject, a possibility of occurrence of hepatocellular carcinoma in a subject, or a risk for future occurrence of hepatocellular carcinoma in a subject. The information on hepatocellular carcinoma in a subject who has already been affected by hepatocellular carcinoma may include prognosis of the subject, a degree of progression (stage) and the like. The information on hepatocellular carcinoma obtained based on the analysis result obtained in the analyzing step does not substantially include information on a type of cancer different from hepatocellular carcinoma because the promoter regions of the above genes which are used as markers in the present method are specifically methylated in cancer cells derived from hepatocellular carcinoma.

In particular embodiments of the present invention, the analyzing step which provides the analysis result indicating the presence of a methylated CpG site may provide information indicating the occurrence of hepatocellular carcinoma or indicating that the status of hepatocellular carcinoma is poor (or aggravated).

In another embodiment of the present invention, the above information can be obtained when the methylation frequency obtained in the analyzing step is higher than a certain threshold.

More specifically, the information obtained is indicative of presence of a cancer cell derived from hepatocellular carcinoma in a biological sample. Also described herein, the information obtained may alternatively indicate that a subject has a high risk for being affected by hepatocellular carcinoma or that a subject has already been affected by hepatocellular carcinoma. For a subject who has already been affected by hepatocellular carcinoma, information obtained may indicate that prognosis of the subject is poor (or aggravated) or that the cancer is in a progressed stage.

When the result from the analyzing step shows absence of methylated CpG site on the contrary, information suggesting no occurrence of hepatocellular carcinoma or information indicating that hepatocellular carcinoma is in a good (or reduced) status can be obtained. Alternatively the above information can be obtained when the methylation frequency obtained in the analyzing step is lower than a certain threshold. More specifically, the information obtained is indicative of the absence of a cancer cell derived from hepatocellular carcinoma in a biological sample. Also described herein, the information obtained may alternatively indicate that a subject has a low risk for being affected by hepatocellular carcinoma or that a subject has not been affected by hepatocellular carcinoma. For a subject who has already been affected by hepatocellular carcinoma, information obtained may be indicative of a positive prognosis of the subject or indicate that the cancer is in a relatively early stage.

The threshold is not particularly limited and may be empirically set based on accumulated data on various biological samples. The threshold may alternatively be set as follows. First, methylation frequency is analyzed for DNA extracted respectively from a biological sample which is confirmed to be devoid of cancer cells derived from hepatocellular carcinoma (normal liver tissue or normal hepatocyte) and a biological sample containing a cancer cell derived from hepatocellular carcinoma. Next, based on the obtained analysis results, a threshold is set within a range that is higher than the methylation frequency of the biological sample devoid of cancer cells and lower than that of the biological sample containing the cancer cell. Preferably, the threshold is set as a value which can highly accurately differentiate between the biological sample devoid of cancer cells and the biological sample containing the cancer cell.

Also described herein is a marker for obtaining information on hepatocellular carcinoma by methylation analysis (also merely referred to as "marker"). The marker is at least one CpG site selected from CpG sites located in promoter regions of CELF6 and RNF135 genes.

Methylation status of the marker in a DNA sample prepared from a biological sample collected from a subject may be analyzed and information on hepatocellular carcinoma in the subject can be obtained based on the analysis result. The analysis of the methylation status and obtainment of information on hepatocellular carcinoma are the same as previously described.

Also described herein is a kit for obtaining information on hepatocellular carcinoma (also merely referred to as "kit"). The kit comprises a primer set for analysis of the methylation status of at least one CpG site selected from CpG sites located in promoter regions of the CELF6 and RNF135 genes.

The primer set in the kit may be a primer set for analysis of the methylation status of CpG sites according to mass spectrometry such as MassARRAY® or an analysis method involving PCR amplification such as the MSP method, the bisulfite sequencing method, among which the primer set for mass spectrometry such as MassARRAY® or for the MSP is preferred. The base sequence of the primers in the primer set may be appropriately selected by a person skilled in the art based on the base sequences in the promoter regions. The primer set used for the MSP method may be a set of primers respectively having base sequences SEQ ID NOs: 3 and 4 or a set of primers respectively having base sequences SEQ ID NOs: 5 and 6.

Also described herein is the use of a polynucleotide obtained by subjecting an isolated DNA to bisulfite treatment, wherein the isolated DNA consists of a base sequence corresponding to the whole or a partial promoter region of CELF6 or RNF135 gene and contains at least one CpG site in the promoter region and at least one cytosine not included in CpG sites (also merely referred to as "polynucleotide") as a marker for obtaining information on hepatocellular carcinoma. The term "cytosine not included in CpG sites" may be any cytosine other than those contained in CpG sites and may include, for example, cytosine in a base sequence in which cytosine (C) and adenine (A), thymine (T) or cytosine (C) are adjacent in this order from 5' to 3' (namely CA, CT or CC).

When the isolated DNA is subjected to bisulfite treatment regarding the polynucleotide as described herein, a non-methylated cytosine in the isolated DNA is converted to uracil while a methylated cytosine is not converted. In particular embodiments of the present invention, information on hepatocellular carcinoma can be obtained by analyzing the methylation status of CpG sites in the polynucleotide. The isolated DNA can be obtained in the same manner as that described for preparation of the DNA sample. The bisulfite treatment, analysis of methylation status and obtainment of information on hepatocellular carcinoma are also the same as previously described.

The polynucleotide of the present invention includes a polynucleotide consisting of a base sequence SEQ ID NO: 9 or 10. The polynucleotide consisting of the base sequence SEQ ID NO: 9 or 10 is suitable for analysis of the methylation status by the MSP method.

Also described herein is a system suitable for provision of information on hepatocellular carcinoma in a subject. The system may be as follows for example.

A system suitable for provision of information on hepatocellular carcinoma in a subject comprising a computer containing a processor and a memory controlled by the processor, wherein the memory comprises a computer program for enabling the computer to carry out a process comprising the steps of:

obtaining an analysis result on the methylation status of a CpG site located in a promoter region of at least one gene selected from CELF6 and RNF135 in a DNA sample derived from the subject; and
providing information on hepatocellular carcinoma in the subject based on the resulting analysis result.

Also described herein is a computer program product for enabling a computer to carry out provision of information on hepatocellular carcinoma in a subject. The computer program product may be as follows for example.

A computer program product for enabling a computer to carry out provision of information on hepatocellular carcinoma in a subject comprising a computer readable medium, wherein the medium comprises a computer program for enabling the computer to carry out a process comprising the steps of:

obtaining an analysis result on methylation status of a CpG site located in a promoter region of at least one gene selected from CELF6 and RNF135 in a DNA sample derived from the subject; and
providing information on hepatocellular carcinoma in the subject based on the resulting analysis result.

The medium may be a non-transitory computer readable medium of the computer program.

A suitable device for carrying out the present method is illustrated by referring to figures. FIG. 8 is a schematic view of an example of a determination device for providing information on hepatocellular carcinoma in a subject. The determination device 1 shown in FIG. 8 comprises a measurement device 2 and a computer system 3 connected to the measurement device 2.

The measurement device 2 may be a MALDI-TOF mass spectrometer. The measurement device 2 obtains mass spectrometric information such as the time of flight or the mass-to-charge ratio (m/z ratio) of a substance to be analyzed. The measurement device 2 onto which a measurement sample prepared from the DNA sample derived from a subject is mounted obtains mass spectrometric information of a nucleic acid in the measurement sample and sends the mass spectrometric information to the computer system 3.

The measurement device 2 may be, when methylation status is analyzed by the MSP method, a gel imaging device such as a fluorescence image scanner. In this case, the measurement device 2 onto which a gel obtained by electrophoresis of a reaction solution after nucleic acid amplification by the MSP method is mounted detects amplification products. The measurement device 2 then obtains the band intensity data of the amplification products and sends the obtained data to the computer system 3.

The computer system 3 comprises a computer main body 3a, an input device 3b and a display 3c for displaying specimen information, determination results and the like. The computer system 3 receives the mass spectrometric information from the measurement device 2. The processor in the computer system 3 executes, based on the mass spectrometric information, a program for providing information on hepatocellular carcinoma in a subject.

FIG. 9 is a block diagram showing the functionality configuration of the determination device of FIG. 8. As shown in FIG. 9, the computer system 3 comprises a receiving unit 301, a memory unit 302, a calculating unit 303, a determining unit 304 and an output unit 305. The receiving unit 301 is communicably connected to the measurement device 2 though a network. The calculating unit 303 and the determining unit 304 are included in a control unit 306.

The receiving unit 301 obtains information from the measurement device 2. The memory unit 302 stores a threshold necessary for determination and a formula for calculating the methylation score. The calculating unit 303 calculates the methylation score from information obtained at the receiving unit 301 according to the formula stored in the memory unit 302. The determining unit 304 determines whether or not the methylation score calculated at the calculating unit 303 is lower than the threshold stored at the memory unit 302. The output unit 305 outputs the determination result from the determining unit 304 as information on hepatocellular carcinoma in the subject (e.g., presence or absence of a cancer cell derived from hepatocellular carcinoma in the biological sample collected from the subject).

FIG. 10 is a block diagram showing the hardware configuration of the determination device in FIG. 8. As shown in FIG. 10, the computer main body 3a comprises a CPU (Central Processing Unit) 30, ROM (Read Only Memory) 121, ROM 32, a hard disk 33, an input/output interface 34, a read-out device 35, a communication interface 36 and an image output interface 37. The CPU 30, ROM 31, RAM (Random Access Memory) 32, the hard disk 33, the input/output interface 34, the read-out device 35, the communication interface 36 and the image output interface 37 are connected via a bus 38 so as to be able to communicate each other.

The CPU 30 can execute a computer program stored in ROM31 and a computer program loaded with ROM 32. When the CPU 30 executes the application program, the functional blocks described above may be executed. Accordingly the computer system serves as a terminal that is a determination device for providing information on hepatocellular carcinoma in a subject.

ROM 31 is made up with mask ROM, PROM, EPROM, EEPROM or the like. ROM 31 stores the computer program executed by the CPU 30 and data used for the execution.

ROM 32 is made up with SRAM, DRAM or the like. ROM 32 is used for readout of the computer programs stored in ROM 31 and the hard disk 33. ROM 32 is also utilized as a work area when the CPU 30 executes these computer programs.

An operating system to be executed by the CPU 30, computer programs such as application programs (the computer program for providing information on hepatocellular carcinoma in a subject) and data for executing the computer programs are installed on the hard disk 33.

The read-out device 35 is made up with a flexible disk drive, a CD-ROM drive, a DVD-ROM drive or the like and can read out the computer program or data stored on a portable memory medium 40.

The input/output interface 34 is made up with a serial interface such as USB, IEEE1394 and RS-232C, a parallel interface such as SCSI, IDE and IEEE1284 and an analog interface formed by a D/A converter and an A/D converter or the like. The input/output interface 34 is connected to the input device 3b such as a keyboard and a mouse. A user can input data into the computer main body 3a by means of the input device 3b.

The communication interface 36 is, for example, an Ethernet® interface. The computer system 3 can send printing data to a printer via the communication interface 36.

The image output interface 37 is connected to the display 3c made up with a LCD, a CRT and the like. Accordingly the display 3c can output an image signal according to image data provided by the CPU 30. The display 3c displays an image (on a screen) according to the input image signal.

The process operations carried out by the determination device 1 for providing information on hepatocellular carcinoma in a subject are illustrated hereinafter. FIG. 11 is a flow chart for providing information on hepatocellular carcinoma using the determination device of FIG. 8. An example is herein illustrated in which mass spectrometric information of a nucleic acid in a measurement sample prepared from a DNA sample derived from a subject is used for calculation of a peak area from which a methylation score is calculated and the methylation score is determined as to whether or not it is lower than a threshold.

In the step S1-1, the receiving unit 301 in the determination device 1 receives from the measurement device 2 mass spectrometric information. In the next step S1-2, the calculating unit 303 calculates from the mass spectrometric information received at the receiving unit 301 a peak area which is sent to the memory unit 302. In the step S1-3, the calculating unit 303 calculates the methylation score based on the peak area stored in the memory unit 302 according to the formula stored in the memory unit 302.

In the step S1-4, the determining unit 304 determines whether or not the methylation score calculated at the calculating unit 303 is lower than the threshold stored in the memory unit 302. When the methylation score is lower than the threshold, the determining unit 304 in the step S1-5 sends a determination result indicating that the biological sample collected from the subject does not contain a cancer cell derived from hepatocellular carcinoma to the output unit 305. When the methylation score is not lower than the threshold (i.e., the methylation score is at or higher than the threshold), the determining unit 304 sends in the step S1-6 a determination result indicating that the biological sample collected from the subject contains a cancer cell derived from hepatocellular carcinoma to the output unit 305.

In the step S1-7, the output unit 305 outputs the determination result as information on hepatocellular carcinoma in the subject so that the display 3c displays the result and/or the printer prints out the result. Accordingly, the determination device can provide, to a physician or the like, information assisting the physician or the like to judge whether or not the subject has hepatocellular carcinoma.

The present invention is specifically described hereinafter by way of Examples which do not limit the present invention.

## Examples

### Reference Example 1: Identification of novel markers from genomic DNA of hepatocellular carcinoma tissues

#### (1) Biological samples

In the present Reference Example, the biological samples used were clinical specimens obtained from patients by hepatectomy. The clinical specimens contained cancerous tissues of hepatocellular carcinoma (99 specimens), non-cancerous tissues of hepatocellular carcinoma (19 specimens) and normal liver tissues (2 specimens). The tissues were immediately frozen with liquid nitrogen after collection and stored at -80°C prior to use.

#### (2) DNA extraction and bisulfite treatment

Genomic DNA was extracted with a QIAamp DNA Mini Kit (QIAGEN) from the above tissues. The obtained DNA (500 ng) was subjected to bisulfite treatment with an EZ DNA Methylation Kit (Zymo Research) and the genomic DNA after treatment was dissolved in sterilized distilled water (10 μl). The genomic DNA contained in the obtained DNA solution (4 μl) was fragmented with a Bioruptor (COSMO BIO).

#### (3) Identification of novel markers

The fragmented genomic DNA was subjected to Infinium Methylation Assay using an Infinium HumanMethylation450 BeadChip (Illumina) in order to search novel markers which are specifically methylated in cancerous tissues of hepatocellular carcinoma. The specific procedures carried out followed the manual attached to the chip.

The Infinium HumanMethylation450 BeadChip includes probes that allow quantification of methylation status of 482,421 CpG sites on human genome. The probes are designed to allow quantification of signal intensity of methylated DNA fragments and non-methylated DNA fragments for respective CpG sites by means of hybridization and single base extension reaction. The 265,824 probes corresponding to about 55% of all probes target the regions within 5 kb from the transcription initiation sites of genes (10.1 probes per gene in average). In the present Reference Example, the signal intensity (signal M) from probes for methylated CpG sites and the signal intensity (signal U) from probes for non-methylated CpG sites were detected on BeadArray Reader and the methylation rate (mCpG) of CpG sites in the respective genes was calculated according to the following calculation formula:

$$(\text{mCpG}) = (\text{signal M})/\{(\text{signal M}) + (\text{signal U})\}$$

The threshold was set as "0.4" for the methylation rate (mCpG) of genes, and a specimen having a methylation rate at or higher than the threshold was designated as "methylation positive specimen". Based on the number of methylation positive specimens, methylation positive rate (%) for genes in various tissues was calculated according to the following formula:

$$\text{Methylation positive rate (\%)} = (\text{number of methylation positive specimens/total number of specimens}) \times 100$$

For example, for cancerous tissue specimens, the methylation positive rate of a gene can be calculated by "(number of methylation positive specimens among cancerous tissue specimens/total number of cancerous tissue specimens) × 100". The gene which showed a statistically significant difference between cancerous tissue specimens and non-cancerous tissue specimens or normal tissue specimens was identified as a marker which is methylated in cancerous tissues.

As a result, promoter regions of the CELF6 and RNF135 genes were identified as markers which are highly methylated in cancerous tissues of hepatocellular carcinoma (see FIGs. 1(A) and 1(B)). These markers may also be referred to as the present markers hereinbelow.

Reference Example 2: Comparison of methylation data between cancer/tumor tissue specimens derived from several types of cancer/tumor, non-cancerous tissue specimens and normal tissue specimens

(1) Collection of methylation data

In the present Reference Example, methylation data of 7 types of cancer/tumor tissue specimens, 7 types of non-cancerous tissue specimens and 19 types of normal tissue specimens were compared. The number of specimens for the respective tissues is shown in the following tables.

Table 2

| Cancer/tumor tissue specimens | |
|---|---|
| Tissue | No. of specimens |
| Brain tumor (brain) | 114 |
| Breast cancer (Breast) | 548 |
| Squamous cell lung cancer (Lung) | 150 |
| Liver cancer (Liver) | 99 |
| Colon cancer (Colon) | 324 |
| Uterine body cancer (Uterus) | 334 |
| Renal clear cell carcinoma (Kidney) | 282 |

Table 3

| Non-cancerous tissue | |
|---|---|
| Tissue | No. of specimens |
| Brain tumor (brain) | 2 |
| Breast cancer (Breast) | 98 |
| Squamous cell lung cancer (Lung) | 43 |
| Liver cancer (Liver) | 19 |
| Colon cancer (Colon) | 40 |
| Uterine body cancer (Uterus) | 36 |
| Renal clear cell carcinoma (Kidney) | 164 |

Table 4

| Tissue | RCAST | Literature 1 | Literature 2 | Total |
|---|---|---|---|---|
| Normal brain (Brain) | 2 | 1 | 0 | 3 |
| Normal oral cavity (Oral) | 2 | 0 | 0 | 2 |
| Normal lung (Lung) | 0 | 2 | 0 | 2 |
| Normal colonic mucosa (Colon) | 2 | 0 | 0 | 2 |
| Normal liver (Liver) | 2 | 0 | 0 | 2 |
| Peripheral blood from healthy subjects (Blood) | 2 | 2 | 0 | 4 |
| Normal skeletal muscle (Skeletal) | 2 | 2 | 0 | 4 |
| Normal testis (Testis) | 1 | 0 | 0 | 1 |
| Normal gastric mucosa (Stomach) | 0 | 1 | 0 | 1 |
| Normal pancreas (Pancreas) | 0 | 2 | 0 | 2 |
| Normal spleen (Spleen) | 0 | 2 | 0 | 2 |
| Normal kidney (Kidney) | 0 | 0 | 0 | 0 |
| Normal adrenal gland (Adrenal gland) | 0 | 2 | 0 | 2 |
| Normal ureter (Ureter) | 0 | 2 | 0 | 2 |
| Normal bladder (Bladder) | 0 | 2 | 0 | 2 |
| Normal lymph nodes (Lymph nodes) | 0 | 2 | 0 | 2 |
| Normal adipose tissue (Adipose tissue) | 0 | 2 | 0 | 2 |
| Normal heart (Heart) | 0 | 1 | 0 | 1 |
| Various normal blood cell components (WB, PBMC, Gran, CD4+, CD8+, CD14+, CD19+, CD56+, Neu, Eos) | 0 | 0 | 60 | 60 |

In Table 4, the methylation data for the specimens indicated in the column "RCAST" were obtained by the present inventors in the similar manner as Reference Example 1 according to Infinium Methylation Assay using Infinium HumanMethylation450 BeadChip (Illumina). The methylation data for the specimens indicated in the columns "Literature 1" and "Literature 2" were the methylation data published in the literature documents cited below obtained with Infinium HumanMethylation450 BeadChip (Illumina). The methylation data in this context are the methylation rate (mCpG) of CpG sites in CELF6 and RNF135 obtained as described in section (3) in Reference Example 1. The average of the positive rate was plotted for the 18 types of normal tissues excluding the normal blood cell components. For normal blood cell components, the average of 60 samples of the respective blood cell components from healthy subjects (6 subjects × 10) was plotted.

- Literature 1: Nazor KL et al., Recurrent variations in DNA methylation in human pluripotent stem cells and their differentiated derivatives. Cell Stem Cell 2012; 10(5): 620-634
- Literature 2: Reinius LE et al., Differential DNA Methylation in Purified Human Blood Cells: Implications for Cell Lineage and Studies on Disease Susceptibility, PLoS One, 7(7) e41361

(2) Comparison of methylation positive rate between cancer/tumor tissue specimens derived from several types of cancer/tumor, non-cancerous tissue specimens and normal tissue specimens.

The threshold was set as "0.4" for the methylation rate (mCpG) of genes, and a specimen having a methylation rate at or higher than the threshold was designated as "methylation positive specimen". Based on the number of methylation positive specimens, methylation positive rate (%) for the present markers in various tissues was calculated according to the above formula. The results are shown in FIGs. 2(A) and 2(B). In FIG. 2, "Normal tissues" represent, among the tissues indicated in Table 4, the normal tissues excluding 60 specimens of various normal blood cell components and "Normal blood cells" represent the 60 specimens of various normal blood cell components.

FIGs. 2(A) and 2(B) show that all present markers are rarely methylated in other types of cancer or human normal tissues or human normal blood cells and thus are specifically and highly methylated in hepatocellular carcinoma.

Comparative Example 1: Comparison of methylation positive rate between cancer/tumor tissue specimens derived from several types of cancer/tumor, non-cancerous tissue specimens and normal tissue specimens.

The methylation positive rate was calculated for CDH1, CDKN2A and GSTP1 genes (hereinafter referred to as "known markers") which have already been known to be methylated in cancer cells derived from hepatocellular carcinoma in the similar manner as Reference Example 2 in the respective tissues. The results are shown in FIGs. 3(A) to 3(C).

According to FIGs. 3(A) and 3(C), CDH1 and GSTP1 showed high positive rates in hepatocellular carcinoma, while methylation thereof was also detected in other types of cancer and in non-cancerous tissues, and thus CDH1 and GSTP1 have low specificity to hepatocellular carcinoma. According to FIG. 3(B), methylation of CDKN2A was detected in other types of cancer and thus CDKN2A has low sensitivity and specificity. Thus known markers have issues in terms of performance as diagnostic markers of hepatocellular carcinoma. Namely it was shown that a search for markers is required to be carried out thereby taking the sensitivity to hepatocellular carcinoma and the specificity in other types of cancer and other non-cancerous tissues into account.

Example 1: Comparison of methylation data between normal liver tissues, hepatic cirrhosis tissues and hepatocellular carcinoma tissues

(1) Biological samples

In the present Example, the biological samples used were cancerous tissues (6 specimens) collected from hepatocellular carcinoma patients. The control samples used were normal liver tissues (2 specimens) and non-cancerous tissues (5 specimens) of hepatocellular carcinoma collected from hepatocellular carcinoma patients.

(2) Preparation of measurement samples and control samples

(i) Extraction of genomic DNA

Genomic DNA was extracted from the above tissues with QIAamp DNA Mini Kit (QIAGEN). The genomic DNA contained in the obtained DNA solution was fragmented with Bioruptor (COSMO BIO). The control genomic DNA used was genomic DNA of human peripheral blood lymphocytes. The genomic DNA from human peripheral blood lymphocytes was amplified with GenomiPhi v2DNA amplification kit (GE Healthcare Life Sciences). The obtained amplification product consisted of non-methylated DNA. The amplification product was fragmented with Bioruptor (COSMO BIO) to obtain a solution of non-methylated DNA fragments (0% methylated DNA). A portion of the non-methylated DNA fragments was subjected to reaction with SssI methylase (New England Biolab) to methylate all cytosines in CG sequences and obtain a solution of methylated DNA fragments (100% methylated DNA).

(ii) Bisulfite treatment

The respective DNA fragments (500 ng) obtained as above were subjected to bisulfite treatment with EZ DNA Methylation Kit (Zymo Research) and the treated genomic DNA was dissolved in sterilized distilled water (80 μl).

(3) Methylation-specific PCR (MSP)

MSP was carried out on the measurement samples and control samples (DNA after bisulfite treatment) obtained in the above section (2). The composition of PCR reagents, primer sets and reaction conditions for PCR in MSP are shown below.

<PCR reagent>

| | |
|---|---|
| DDW (sterilized water) | 16.8 μL |
| 10 × PCR buffer with $MgCl_2$ (Roche) | 2.5 μL |
| 2 mM dNTP mix | 2.5 μL |
| 10 μM sense primer | 1.0 μL |
| 10 μM anti-sense primer | 1.0 μL |

(continued)

| | |
|---|---|
| Faststart Taq polymerase (Roche) | 0.2 μL |
| Measurement sample or control sample | 1.0 uL |
| Total | 25 μL |

<Primer set>

The primer sets used for MSP are shown in Table 5. These primer sets allow generation of amplification products when DNA in the target regions is methylated (hereinafter also referred to as "primer set for methylation detection"). A primer set for accuracy control was also used which allows judgment on whether or not the bisulfite treatment has been appropriately carried out (see Table 6). The base sequences of bisulfite-converted regions which are amplified with the primer sets for methylation detection for CELF6 and RNF135 are shown in SEQ ID NOs: 9 and 10, respectively. The base sequences shown in SEQ ID NOs: 9 and 10 respectively represent the regions amplified with the respective primer sets for methylation detection with all CpG sites located therein being methylated.

Table 5

| Gene Name | Primer name | Base sequence | SEQ ID NO: | Size of PCR product (bp) | Annealing temp. (°C) (X) | Cycles (Y) | Amplified gene region |
|---|---|---|---|---|---|---|---|
| CELF6 | CELF6_MF | TTTTCGAATGTTTTGTGTGTGTC | 3 | 129 | 58 | 36 | chr15:72612670-72612814 |
| | CELF6_MR | AAAAATTAACCCCGACTCC | 4 | | | | |
| RNF135 | RNF135_MF | GTTGGTCGAGGACGATTTC | 5 | 89 | 58 | 36 | chr17:29298128-29298222 |
| | RNF135_MR | AACAATAACGACAAAAACTATAA | 6 | | | | |

Table 6

| Base sequence of primers | | SEQ ID NO: | Size of PCR product (bp) | Annealing temp. (°C) | Cycles |
|---|---|---|---|---|---|
| Forward | GGGATATTAAGTGGAGTTATTTGGTTTTAGTT | 7 | 129 | 60 | 36 |
| Reverse | CCCTCCCAACATCCTTCCTAA | 8 | | | |

<PCR reaction conditions>

95°C for 6 minutes;
Y cycles of 95°C for 30 seconds, X°C for 30 seconds and 72°C for 30 seconds;
72°C for 7 minutes; and
keep at 16°C.

In the above reaction conditions, "X" and "Y" respectively represent the annealing temperature and the number of cycles as indicated in Tables 5 and 6.

(4) Analysis of results of methylation-specific PCR (MSP)

The amplification product from MSP was verified by 2% agarose gel electrophoresis. The results are shown in FIG. 4. In this figure, "0" and "100" under "control" represent the 0% methylation control sample and the 100% methylation control sample, respectively.

In PCR using the primer set for accuracy control, bands were detected for all samples (data not shown). This shows that bisulfite treatment of the samples was appropriately carried out. In PCR using the primer sets for methylation detection, bands derived from methylated CpGs were not detected for any normal liver tissues or non-cancerous tissues. In contrast, PCR of hepatocellular carcinoma tissue samples resulted in detection of bands in 5 samples among 6 samples for both markers of CELF6 and RNF135. Accordingly it is indicated that in methylation analysis of the present markers by MSP method, methylation of the present markers and hepatocellular carcinoma are correlated as the result of the Infinium method from Reference Example 1. Namely it is suggested that CELF6 and RNF135 are markers with high specificity such that they are highly methylated in hepatocellular carcinoma but methylation thereof is not detected in normal tissues and non-cancerous tissues.

Example 2: Comparison of methylation data between plasma from healthy subjects and plasma from hepatocellular carcinoma patients

(1) Biological samples

The biological samples used in the present Example were peripheral blood (6 specimens) obtained from hepatocellular carcinoma patients. The control samples used were peripheral blood (3 specimens) collected from healthy subjects.

(2) Preparation of measurement samples and control samples

(i) Extraction of genomic DNA

Plasma was obtained from peripheral blood (2 ml) according to the conventional method. Genomic DNA was extracted from the plasma with QIAamp Circulating Nucleic Acid Kit (QIAGEN). The genomic DNA for control used was genomic DNA of human peripheral blood lymphocytes. In the same manner as in Example 1, a solution of the non-methylated DNA fragments (0% methylated DNA) and a solution of methylated DNA fragments (100% methylated DNA) were obtained from the genomic DNA of human peripheral blood lymphocytes.

(ii) Bisulfite treatment

The respective DNA fragments (500 ng) obtained as above were subjected to bisulfite treatment with EZ DNA Methylation Kit (Zymo Research) and the treated genomic DNA was dissolved in sterilized distilled water (80 μl).

(3) MSP

MSP was carried out on the measurement samples and control samples (DNA after bisulfite treatment) obtained in the above section (2). The PCR reagent and primer sets used for MSP are the same as Example 1. The reaction conditions for PCR are shown below.

<PCR reaction conditions>

95°C for 6 minutes;
50 cycles of 95°C for 30 seconds, 60°C for 30 seconds and 72°C for 30 seconds;
72°C for 7 minutes; and
keep at 16°C.

(4) Analysis of results of MSP

The amplification product from MSP was verified by 2% agarose gel electrophoresis. The results are shown in FIG. 5. In this figure, "0" and "100" under "control" represent the 0% methylation control sample and the 100% methylation control sample, respectively.

In PCR using the primer set for accuracy control, bands were detected for all samples (data not shown). This reveals that bisulfite treatment of the samples was appropriately carried out. In PCR using the primer sets for methylation detection, bands derived from methylated CpGs were not detected for plasma samples from healthy subjects. In contrast, PCR of plasma samples from hepatocellular carcinoma patients resulted in detection of bands in 4 samples among 6 samples for CELF6 and 5 samples among 6 samples for RNF135. Accordingly it is indicated that in methylation analysis of the present markers by MSP method on plasma samples, methylation of the present markers and hepatocellular carcinoma are correlated as the result of the Infinium method from Reference Example 1. Namely it is suggested that CELF6 and RNF135 are markers with high specificity such that they are highly methylated in plasma of hepatocellular carcinoma patients but methylation thereof is not detected in plasma from healthy subjects.

Example 3: Comparison of methylation data between cancer tissues other than hepatocellular carcinoma and normal tissues

(1) Biological samples

The biological samples used in the present Example were tissue samples collected from patients with cancer other than hepatocellular carcinoma and various normal organ tissues. The details of the samples are shown in Table 7. In Table 7, "T" represents a tumor tissue, "N" represents a normal tissue and "PBLN" represents peribronchial lymph node.

Table 7

<table>
<tr><th colspan="2">Original tissue</th><th>No. of specimens</th><th>Source</th><th>Symbol</th></tr>
<tr><td rowspan="5">Colon</td><td rowspan="2">Cancer</td><td rowspan="2">2</td><td rowspan="5">Purchased from BioChain Institute, Inc.</td><td>T1</td></tr>
<tr><td>T2</td></tr>
<tr><td rowspan="3">Normal</td><td rowspan="3">3</td><td>N1</td></tr>
<tr><td>N2</td></tr>
<tr><td>N3</td></tr>
<tr><td rowspan="3">Lung</td><td>Cancer</td><td>1</td><td>Purchased from BioChain Institute, Inc.</td><td>T</td></tr>
<tr><td rowspan="2">Normal</td><td rowspan="2">2</td><td rowspan="2">The University of Tokyo</td><td>N1</td></tr>
<tr><td>N2</td></tr>
<tr><td rowspan="3">Mammary gland</td><td>Cancer</td><td>1</td><td rowspan="3">Purchased from BioChain Institute, Inc.</td><td>T</td></tr>
<tr><td rowspan="2">Normal</td><td rowspan="2">2</td><td>N1</td></tr>
<tr><td>N2</td></tr>
</table>

(continued)

| Original tissue | | No. of specimens | Source | Symbol |
|---|---|---|---|---|
| Kidney | Cancer | 1 | Purchased from BioChain Institute, Inc. | T |
| | Normal | 2 | The University of Tokyo | N1 |
| | | | | N2 |
| Corpus uteri | Cancer | 1 | The University of Tokyo | T |
| | Normal | 2 | | N1 |
| | | | | N2 |
| Bladder | Cancer | 1 | Purchased from BioChain Institute, Inc. | T |
| | Normal | 1 | | N |
| Cervix uteri | Normal | 1 | Purchased from BioChain Institute, Inc. | Cervix |
| Testis | Normal | 1 | Purchased from BioChain Institute, Inc. | Testis |
| Blood cell | Normal | 1 | Purchased from BioChain Institute, Inc. | PBLN |
| Stomach | Cancer | 1 | Purchased from BioChain Institute, Inc. | T |
| | Normal | 1 | | N |

(2) Preparation of measurement samples and control samples

(i) Extraction of genomic DNA

Genomic DNA was extracted from the respective tissue samples in the similar manner as Example 1. The genomic DNA for control used was genomic DNA of human peripheral blood lymphocytes. In the same manner as in Example 1, a solution of the non-methylated DNA fragments (0% methylated DNA) and a solution of methylated DNA fragments (100% methylated DNA) were obtained from the genomic DNA of human peripheral blood lymphocytes.

(ii) Bisulfite treatment

The respective DNA fragments (500 ng) obtained as above were subjected to bisulfite treatment with EZ DNA Methylation Kit (Zymo Research) and the treated genomic DNA was dissolved in sterilized distilled water (80 μl).

(3) MSP

MSP was carried out on the measurement samples and control samples (DNA after bisulfite treatment) obtained in the above section (2). The primer sets used for MSP in the present Example are the same as those used in Example 1. The composition of the PCR reagent and PCR reaction conditions are shown below:

<PCR reagent>

| | |
|---|---|
| DW (Sterilized water) | 18.8 μL |
| 10 × PCR buffer with $MgCl_2$ (Roche) | 2.5 μL |
| 10 mM dNTP mix | 0.5 μL |
| 10 μM sense primer | 1.0 μL |
| 10 μM anti-sense primer | 1.0 μL |
| Faststart Taq polymerase (Roche) | 0.2 μL |
| Measurement sample or control sample | 1.0 μL |
| Total | 25 μL |

<PCR reaction conditions>

95°C for 6 minutes;
38 cycles of 95°C for 30 seconds, 58°C for 15 seconds and 72°C for 30 seconds;
72°C for 7 minutes; and
keep at 16°C.

(4) Analysis of results of MSP

The amplification product from MSP was verified by 2% agarose gel electrophoresis. The results are shown in FIG. 6. "NC" and "PC" in the figure respectively represent the 0% methylation control sample and the 100% methylation control sample. "rCpG" represents the amplification product using the primers for accuracy control. The band intensity of the amplification products in FIG. 6 was also measured. The results are shown in FIGs. 7(A) to 7(C).

FIG. 6 shows that in PCR using the primer set for accuracy control, bands were detected for all samples and thus the bisulfite treatment of the samples was appropriately carried out. In PCR using the primer sets for methylation detection for CELF6 and RNF135, bands derived from methylated CpGs were detected for the 100% methylated control sample while no band was detected for other samples. FIGs. 7(A) to 7(C) show that from comparison with the band intensity from PCR using the primer set for accuracy control, the band intensity from PCR using the primer sets for methylation detection of CELF6 and RNF135 corresponded to the background. From these results, it is suggested that the present markers the ones which are not detected so as to be methylated in cancer tissues other than hepatocellular carcinoma or in normal tissues.

**Claims**

1. A method for obtaining information on the presence or absence of a cancer cell derived from hepatocellular carcinoma in the biological sample collected from the subject comprising the steps of:

    preparing a DNA sample from a biological sample collected from a subject;
    analyzing the methylation status of a CpG site in a promoter region of at least one marker gene specific to hepatocellular carcinoma, the marker gene being selected from CELF6 and RNF135 in the DNA sample obtained from the preparation step,
    wherein the analyzing step is the step of analyzing the presence or absence of methylation of at least one CpG site in the promoter region corresponding to a region consisting of SEQ ID NO: 9 or 10 wherein U is replaced with C; or analyzing methylation frequency; and
    obtaining information on hepatocellular carcinoma in the subject based on an analysis result obtained from the analyzing step,
    wherein the biological sample contains a cancer cell derived from hepatocellular carcinoma when the analysis result shows that there is a methylated CpG site, or when the methylation frequency obtained from the analyzing step is higher than a predetermined threshold.

2. The method according to claim 1, wherein the analysis result is obtained by using a marker for obtaining information on hepatocellular carcinoma by methylation analysis, which is at least one CpG site selected from CpG sites located in promoter regions of the CELF6 and RNF135 genes.

3. The method according to claim 1, wherein the analysis result is obtained by using a kit comprising a primer set for analysis of methylation of at least one CpG site selected from CpG sites located in promoter regions of the CELF6 and RNF135 genes.

4. The method according to claim 3, wherein the primer set is a primer set for analyzing methylation status of the CpG site by at least one method selected from mass spectrometry and methylation-specific PCR method.

5. The method according to claim 4, wherein the primer set is at least one selected from a primer set of primers respectively having base sequences SEQ ID NOs: 3 and 4 and a primer set of primers respectively having base sequences SEQ ID NOs: 5 and 6.

## Patentansprüche

1. Verfahren zum Gewinnen von Informationen in Bezug auf die Anwesenheit oder Abwesenheit einer Krebszelle, die aus einem hepatozellulären Karzinom in der aus dem Individuum entnommenen biologischen Probe stammt, umfassend die Schritte:

   Herstellen einer DNA-Probe aus einer aus dem Individuum entnommenen biologischen Probe;
   Analysieren des Methylierungszustand einer CpG-Stelle in einer Promotorregion von mindestens einem für hepatozelluläres Karzinom spezifischen Markergen, wobei das Markergen ausgewählt ist aus CELF6 und RNF135 in der aus dem Herstellungsschritt erhaltenen DNA-Probe;
   wobei der Analyseschritt der Schritt des Analysierens der Anwesenheit oder Abwesenheit von Methylierung von mindestens einer CpG-Stelle in der Promotorregion korrespondierend zu ein Region bestehend aus SEQ ID NO: 9 oder 10 wobei U durch C ersetzt ist, oder des Analysierens der Methylierungshäufigkeit ist; und
   Gewinnen von Informationen in Bezug auf das hepatozelluläre Karzinom in dem Individuum, basierend auf einem Analyseergebnis, das aus dem Analyseschritt erhalten wird,
   wobei die biologische Probe eine Krebszelle enthält, die aus einem hepatozellulären Karzinom stammt, wenn das Analyseergebnis zeigt, dass eine methylierte CpG-Stelle vorliegt oder wenn die aus dem Analyseschritt erhaltene Methylierungshäufigkeit höher als ein festgelegter Schwellenwert ist.

2. Verfahren nach Anspruch 1, wobei das Analyseergebnis erhalten wird, indem ein Marker zum Gewinnen von Informationen in Bezug auf hepatozelluläres Karzinom durch Methylierungsanalyse verwendet wird, wobei es sich um mindestens eine CpG-Stelle handelt, die aus CpG-Stellen ausgewählt ist, die sich in Promotorregionen der CELF6- und RNF135-Gene befinden.

3. Verfahren nach Anspruch 1, wobei das Analyseergebnis erhalten wird, indem ein Kit verwendet wird, das einen Primersatz zur Analyse der Methylierung von mindestens einer CpG-Stelle umfasst, die aus CpG-Stellen ausgewählt ist, die sich in Promotorregionen der CELF6- und RNF135-Gene befinden.

4. Verfahren nach Anspruch 3, wobei der Primersatz ein Primersatz zum Analysieren des Methylierungszustand der CpG-Stelle durch mindestens ein Verfahren ausgewählt aus Massenspektrometrie und methylierungsspezifischem PCR-Verfahren ist.

5. Verfahren nach Anspruch 4, wobei der Primersatz mindestens einer ist, ausgewählt aus einem Primersatz von Primern mit den Basensequenzen SEQ ID NO: 3 bzw. 4 und einem Primersatz von Primern mit den Basensequenzen SEQ ID NO: 5 bzw. 6.

## Revendications

1. Procédé destiné à obtenir des informations sur la présence ou l’absence d’une cellule cancéreuse dérivée d’un carcinome hépatocellulaire dans l’échantillon biologique prélevé sur le sujet, comprenant les étapes consistant à :

   préparer un échantillon d’ADN provenant d’un échantillon biologique prélevé sur un sujet ;
   analyser l’état de méthylation d’une région promoteur d’au moins un gène marqueur spécifique d’un carcinome hépatocellulaire, le gène marqueur étant choisi parmi les CELF6 et RNF135 dans l’échantillon d’ADN obtenu à partir de l’étape de préparation,

dans lequel l'étape d'analyse est l'étape d'analyse de la présence ou de l'absence d'une méthylation d'au moins un site CpG dans la région promoteur correspondant à une region constituée par les SEQ ID n° : 9 ou 10 dans lesquels U est remplacé par C, ou d'analyse de la fréquence de méthylation ; et
obtenir des informations sur un carcinome hépatocellulaire chez le sujet, sur la base d'un résultat d'analyse obtenu à partir de l'étape d'analyse,
dans lequel l'échantillon biologique contient une cellule cancéreuse dérivée d'un carcinome hépatocellulaire quand le résultat de l'analyse montre qu'il y a un site CpG méthylé ou quand la fréquence de méthylation obtenue à partir de l'étape d'analyse est supérieure à un seuil prédéterminé.

**2.** Procédé selon la revendication 1, dans lequel le résultat de l'analyse est obtenu en utilisant un marqueur pour obtenir des informations sur un carcinome hépatocellulaire, par une analyse de la méthylation, qui est au moins un site CpG choisi parmi des sites CpG situés dans des régions promoteurs des gènes CELF6 et RNF135.

**3.** Procédé selon la revendication 1, dans lequel le résultat de l'analyse est obtenu en utilisant une trousse qui comprend un ensemble d'amorces destiné à une analyse de la méthylation d'au moins un site CpG choisi parmi des sites CpG situés dans des régions promoteurs des gènes CELF6 et RNF135.

**4.** Procédé selon la revendication 3, dans lequel l'ensemble d'amorces est un ensemble d'amorces destiné à analyser l'état de méthylation du site CpG par au moins un procédé choisi parmi la spectrométrie de masse et un procédé d'ACP spécifique de la méthylation.

**5.** Procédé selon la revendication 4, dans lequel l'ensemble d'amorces en est un au moins choisi parmi un ensemble d'amorces ayant respectivement les séquences de base SEQ ID n° : 3 et 4, et un ensemble d'amorces ayant respectivement les séquences de base SEQ ID n° : 5 et 6.

FIG. 1(A)

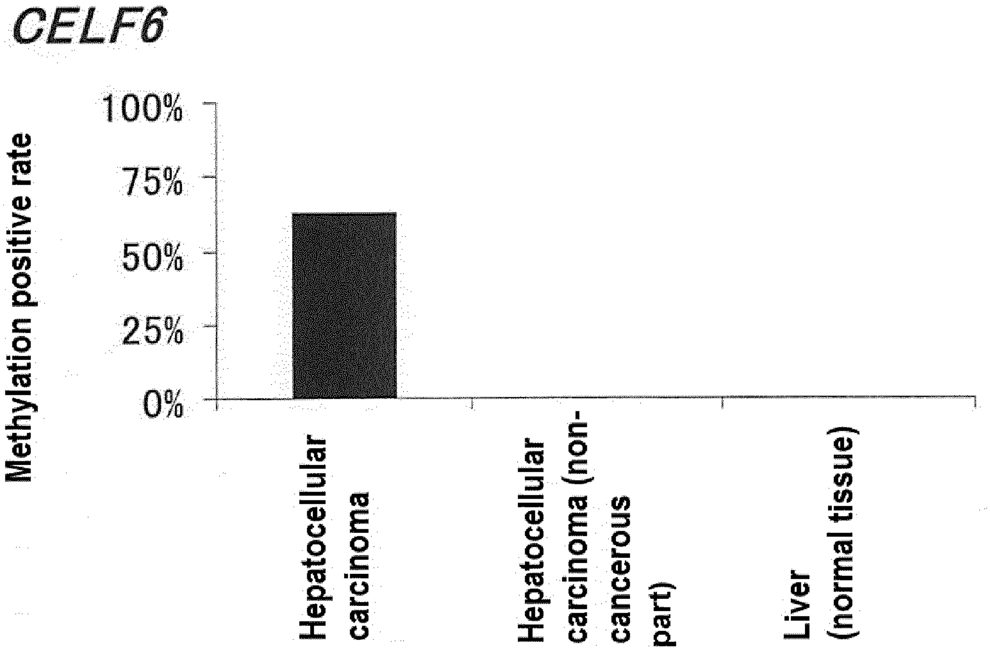
CELF6
Methylation positive rate
100%
75%
50%
25%
0%
Hepatocellular carcinoma
Hepatocellular carcinoma (non-cancerous part)
Liver (normal tissue)

FIG. 1(B)

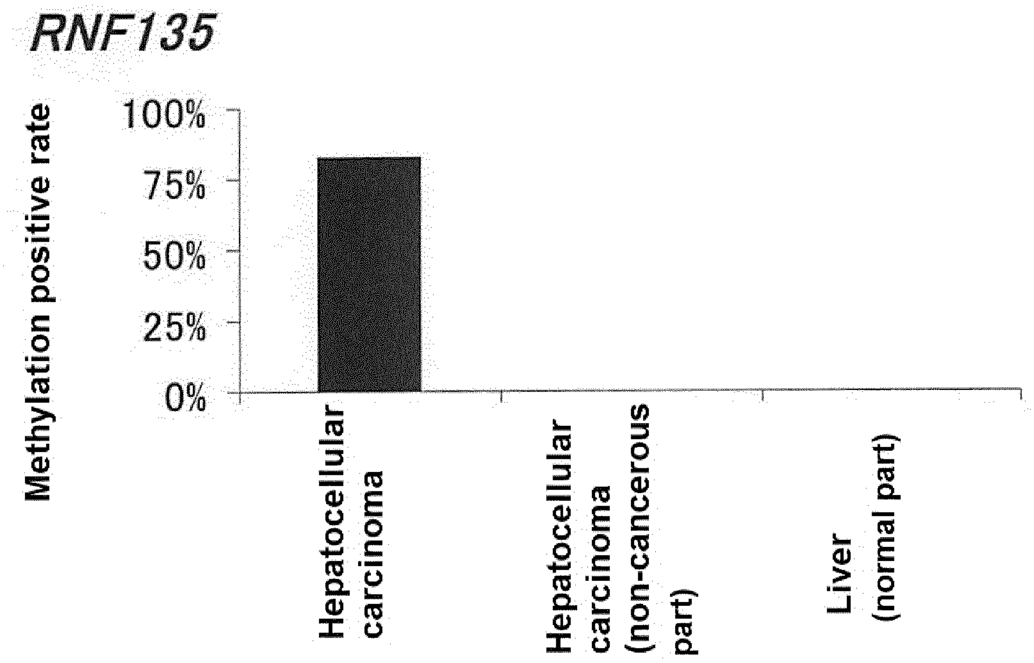
RNF135
Methylation positive rate
100%
75%
50%
25%
0%
Hepatocellular carcinoma
Hepatocellular carcinoma (non-cancerous part)
Liver (normal part)

FIG. 2(A)

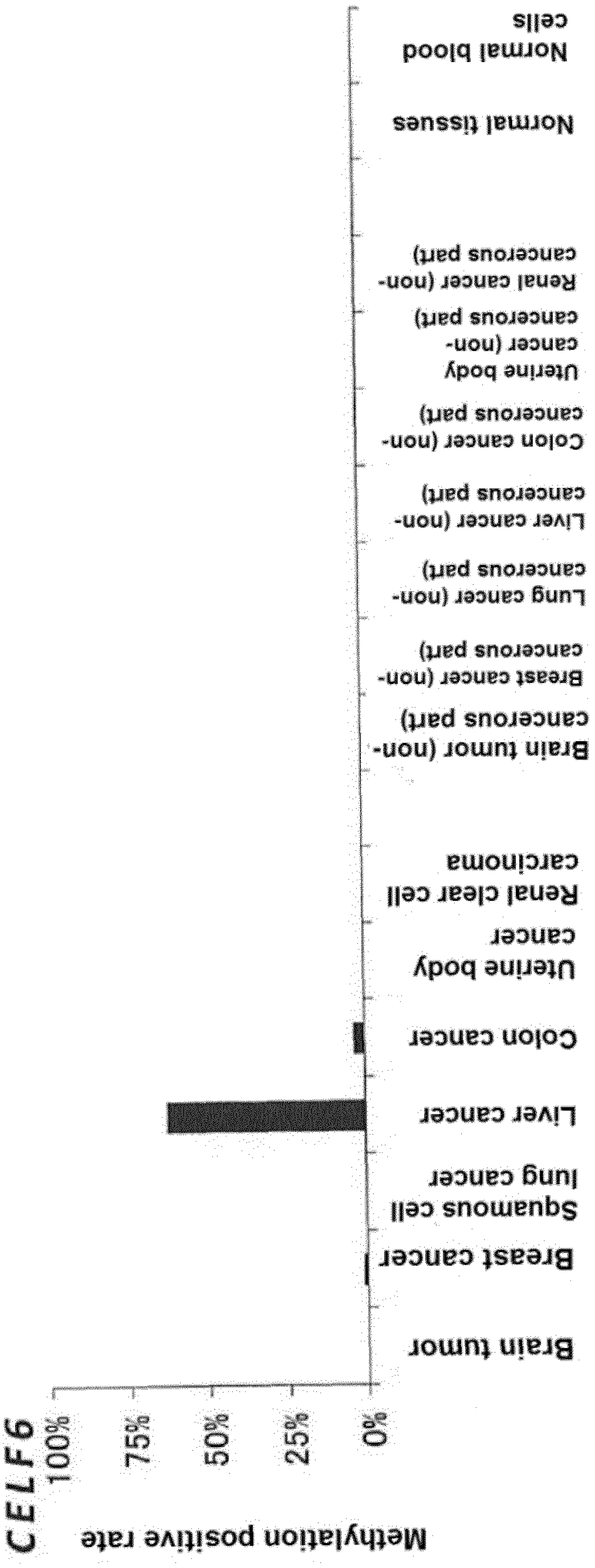
CELF6
Methylation positive rate
100%
75%
50%
25%
0%
Brain tumor
Breast cancer
Squamous cell lung cancer
Liver cancer
Colon cancer
Uterine body cancer
Renal clear cell carcinoma
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous part)
Renal cancer (non-cancerous part)
Normal tissues
Normal blood cells

FIG. 2(B)

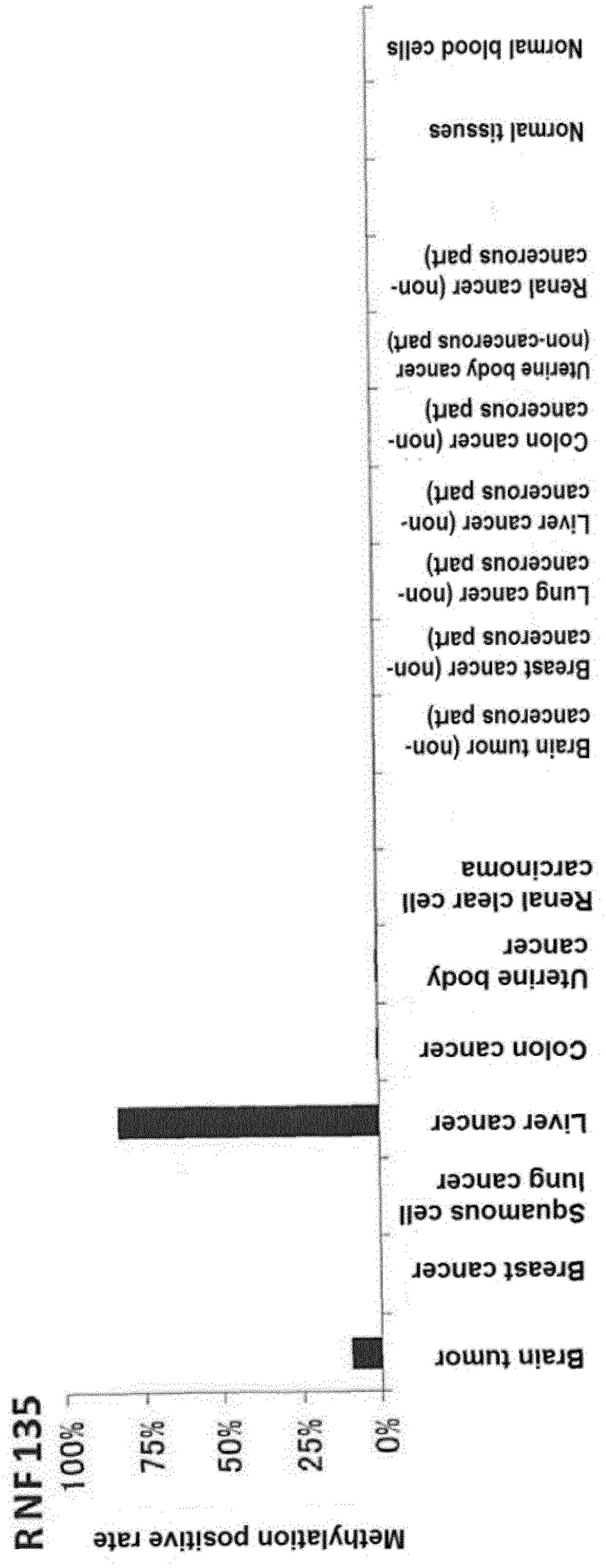
RNF135
Methylation positive rate
100%
75%
50%
25%
0%
Brain tumor
Breast cancer
Squamous cell lung cancer
Liver cancer
Colon cancer
Uterine body cancer
Renal clear cell carcinoma
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous part)
Renal cancer (non-cancerous part)
Normal tissues
Normal blood cells

FIG. 3(A)

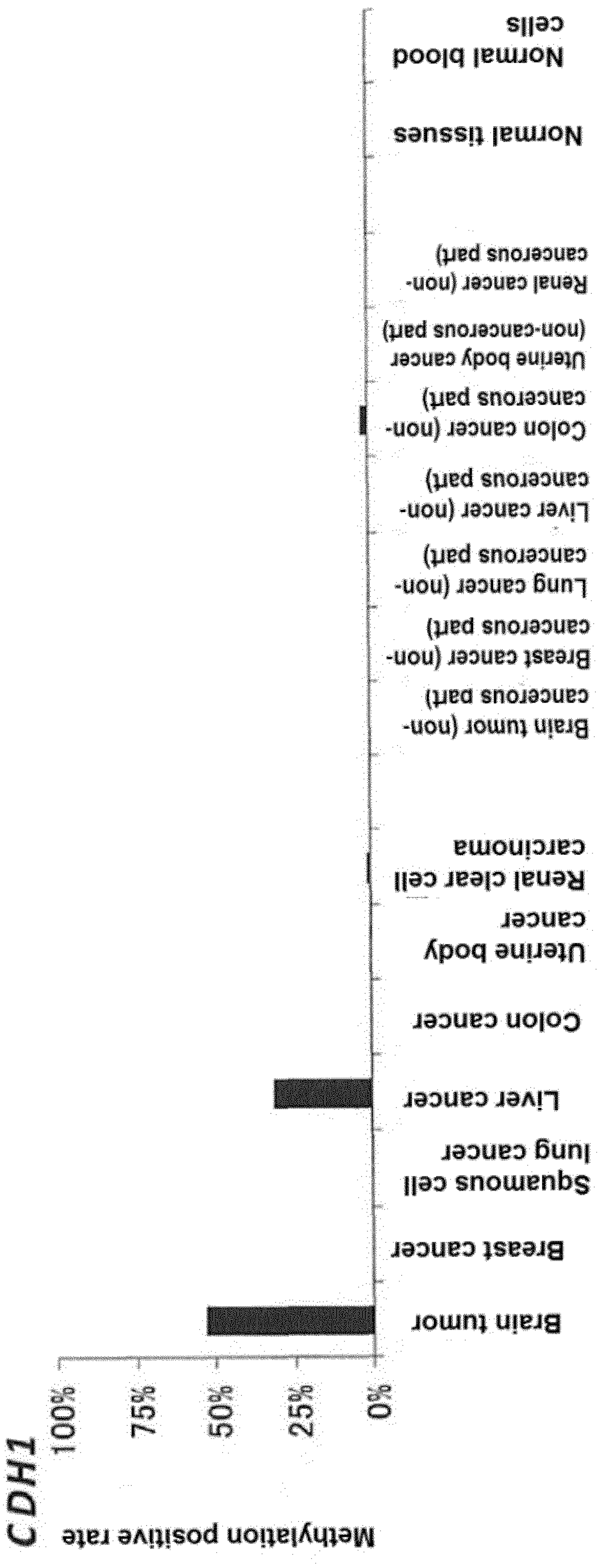
CDH1
Methylation positive rate
100%
75%
50%
25%
0%
Brain tumor
Breast cancer
Squamous cell lung cancer
Liver cancer
Colon cancer
Uterine body cancer
Renal clear cell carcinoma
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous part)
Renal cancer (non-cancerous part)
Normal tissues
Normal blood cells

FIG. 3(B)

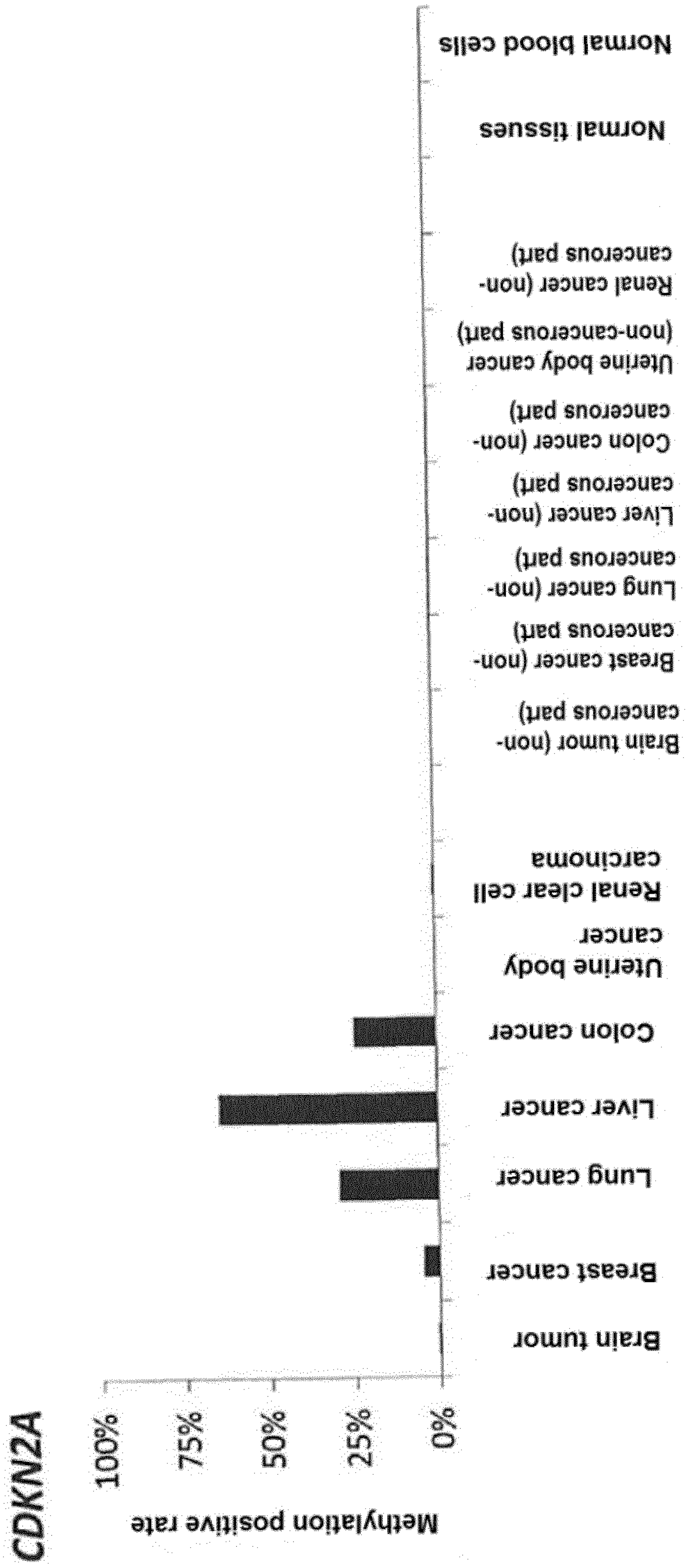
CDKN2A
Methylation positive rate
100%
75%
50%
25%
0%
Brain tumor
Breast cancer
Lung cancer
Liver cancer
Colon cancer
Uterine body cancer
Renal clear cell carcinoma
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous part)
Renal cancer (non-cancerous part)
Normal tissues
Normal blood cells

FIG. 3(C)

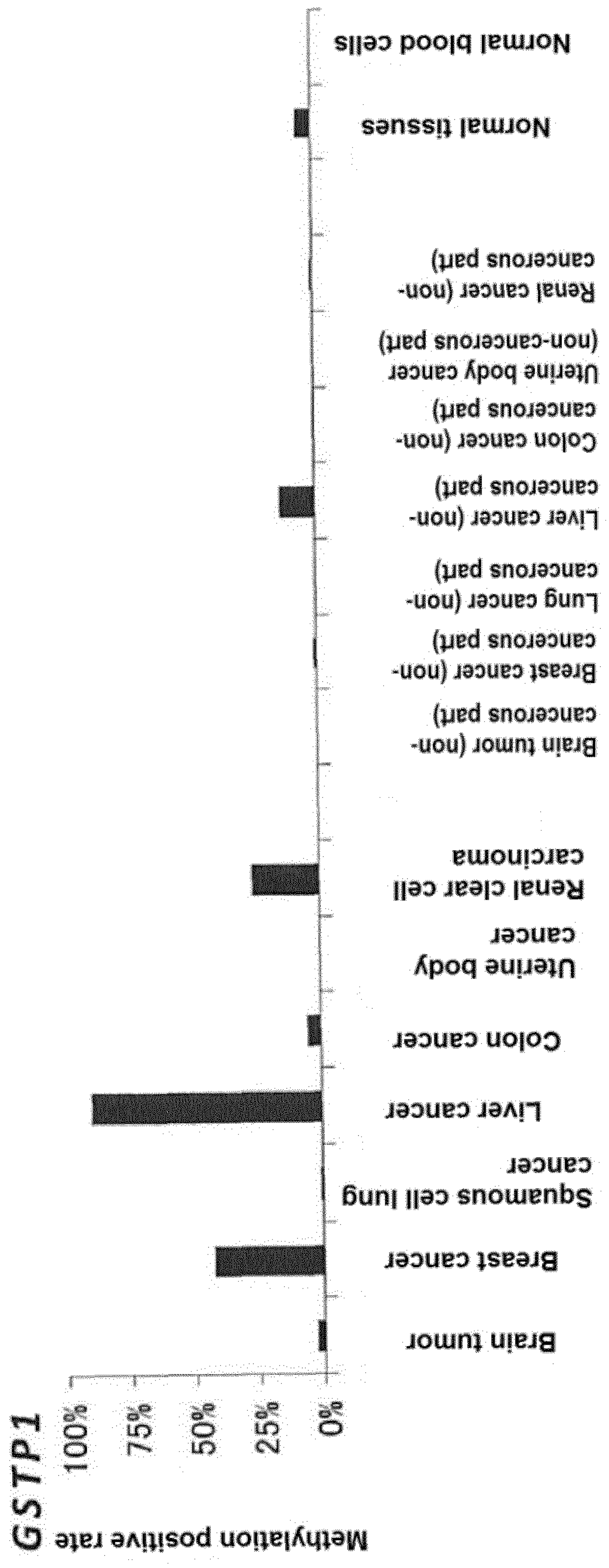
GSTP1
Methylation positive rate
100%
75%
50%
25%
0%
Brain tumor
Breast cancer
Squamous cell lung cancer
Liver cancer
Colon cancer
Uterine body cancer
Renal clear cell carcinoma
Brain tumor (non-cancerous part)
Breast cancer (non-cancerous part)
Lung cancer (non-cancerous part)
Liver cancer (non-cancerous part)
Colon cancer (non-cancerous part)
Uterine body cancer (non-cancerous part)
Renal cancer (non-cancerous part)
Normal tissues
Normal blood cells

FIG. 4

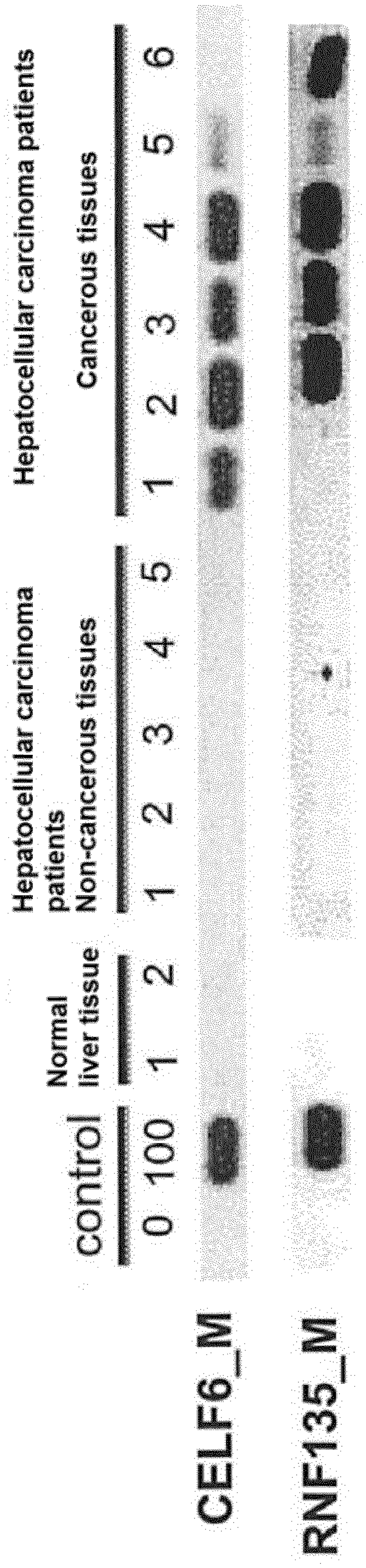
control
0 100
Normal liver tissue
1 2
Hepatocellular carcinoma patients
Non-cancerous tissues
1 2 3 4 5
Hepatocellular carcinoma patients
Cancerous tissues
1 2 3 4 5 6
CELF6_M
RNF135_M

FIG. 5

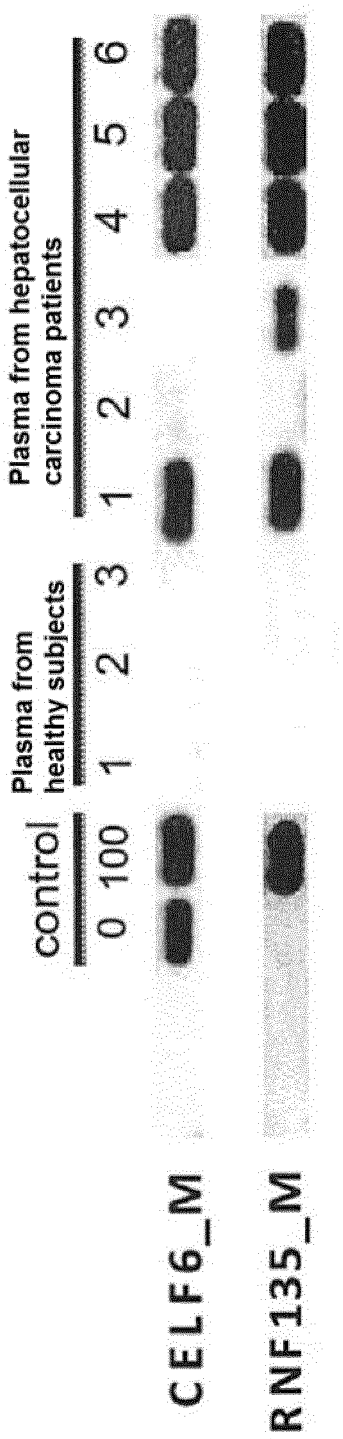
control
0 100
Plasma from healthy subjects
1 2 3
Plasma from hepatocellular carcinoma patients
1 2 3 4 5 6
CELF6_M
RNF135_M

FIG. 6

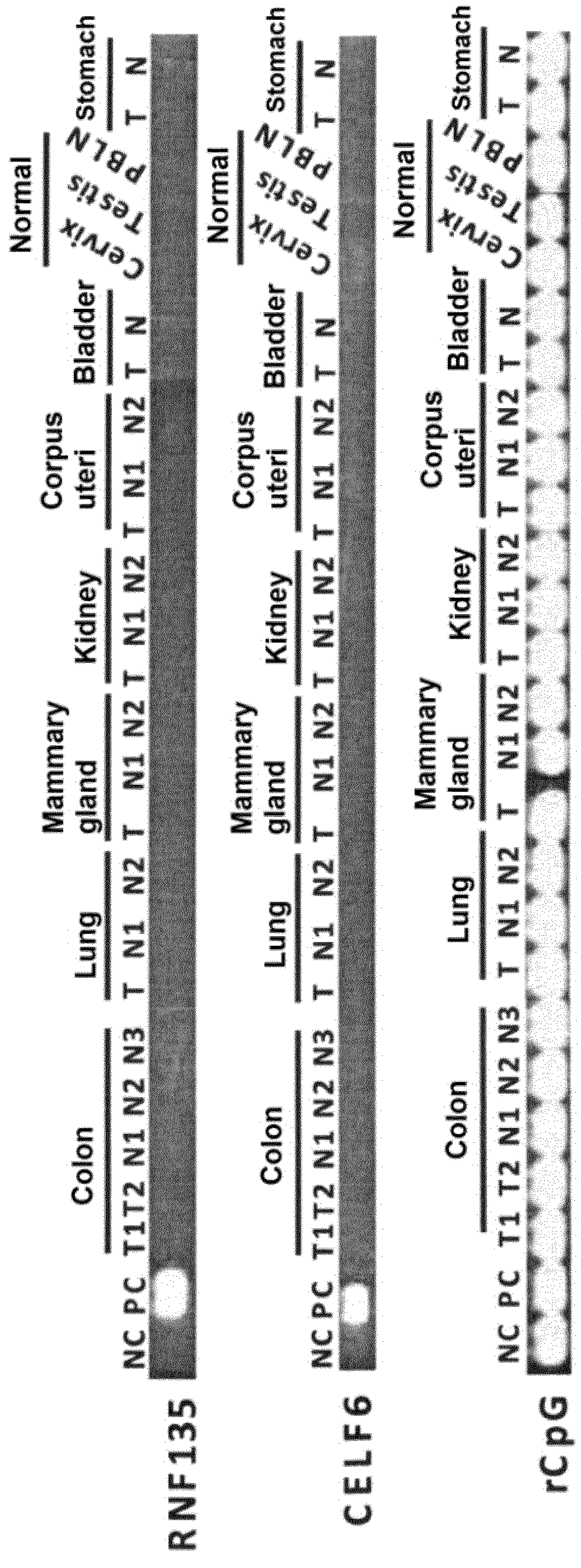
RNF135
CELF6
rCpG
NC PC
Colon
T1 T2 N1 N2 N3
Lung
T N1 N2
Mammary gland
T N1 N2
Kidney
T N1 N2
Corpus uteri
T N1 N2
Bladder
T N
Normal
Cervix Testis PBL N
Stomach
T N

FIG. 7(A)

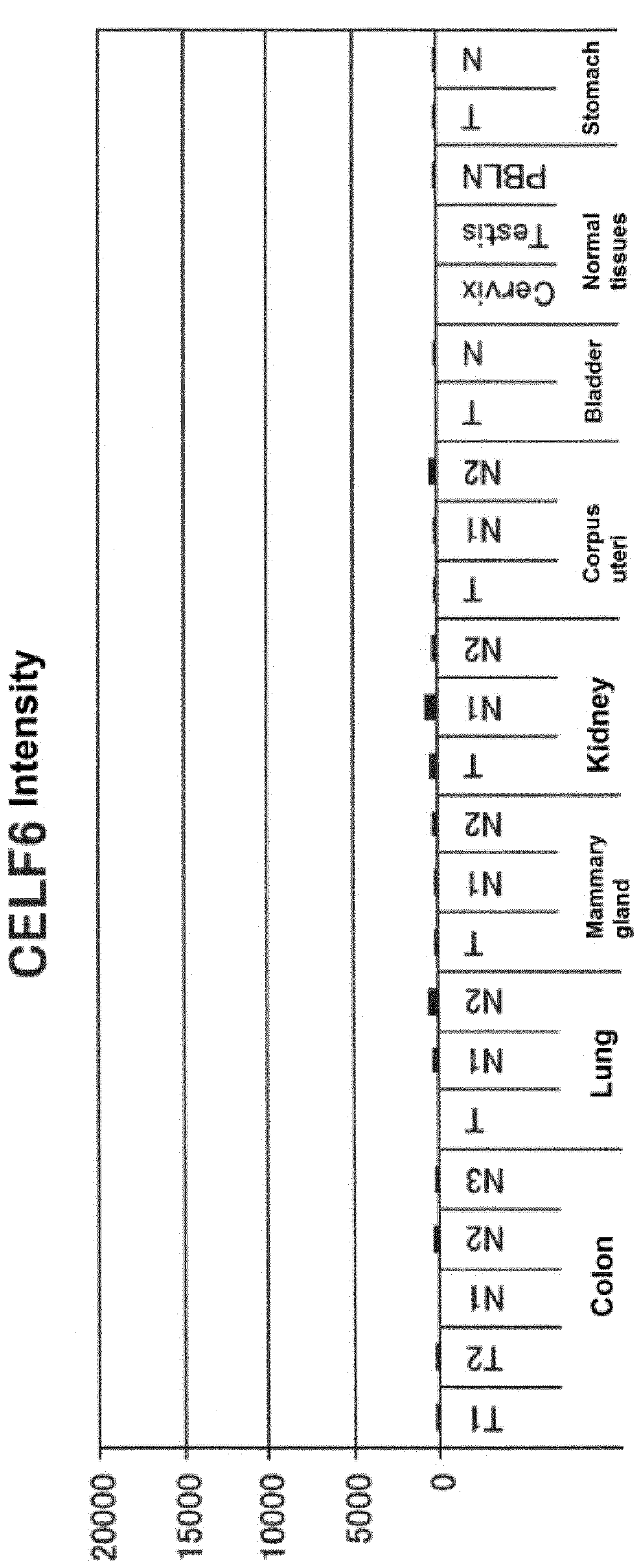
CELF6 Intensity
20000
15000
10000
5000
0
T1
T2
N1
N2
N3
Colon
T
N1
N2
Lung
T
N1
N2
Mammary gland
T
N1
N2
Kidney
T
N1
N2
Corpus uteri
T
N
Bladder
Cervix
Testis
PBLN
Normal tissues
T
N
Stomach

FIG. 7(B)

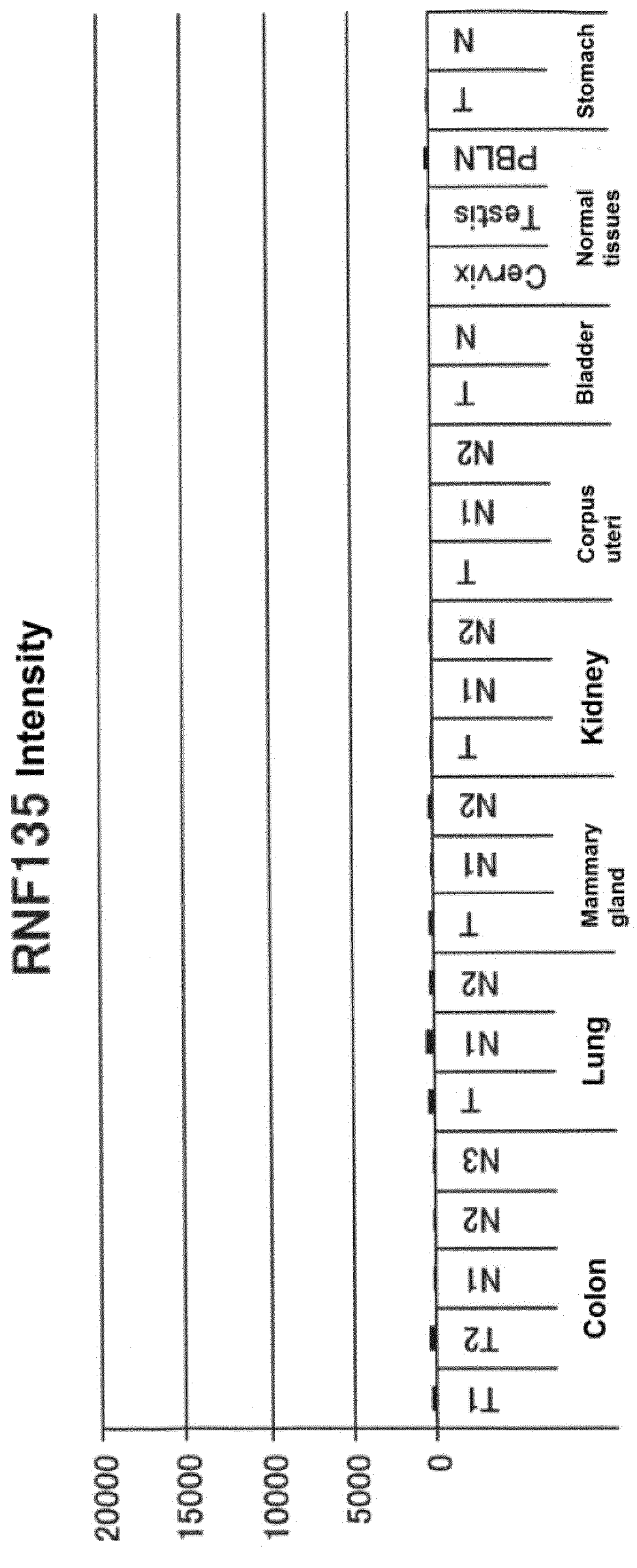
RNF135 Intensity
20000
15000
10000
5000
0
T1
T2
N1
N2
N3
Colon
T
N1
N2
Lung
T
N1
N2
Mammary gland
T
N1
N2
Kidney
T
N1
N2
Corpus uteri
T
N
Bladder
Cervix
Testis
PBLN
Normal tissues
T
N
Stomach

FIG. 7(C)

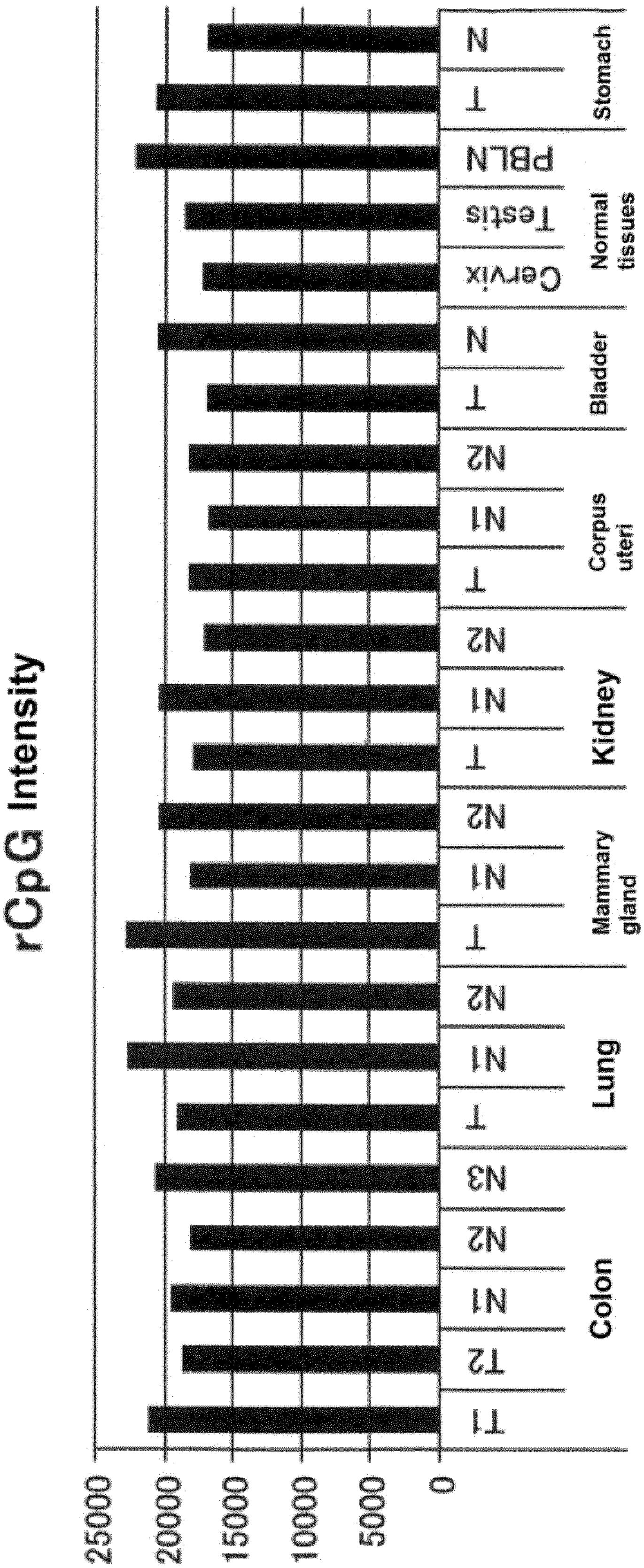
rCpG Intensity
25000
20000
15000
10000
5000
0
T1
T2
N1
N2
N3
Colon
T
N1
N2
Lung
T
N1
N2
Mammary gland
T
N1
N2
Kidney
T
N1
N2
Corpus uteri
T
N
Bladder
Cervix
Testis
PBLN
Normal tissues
T
N
Stomach

FIG. 8

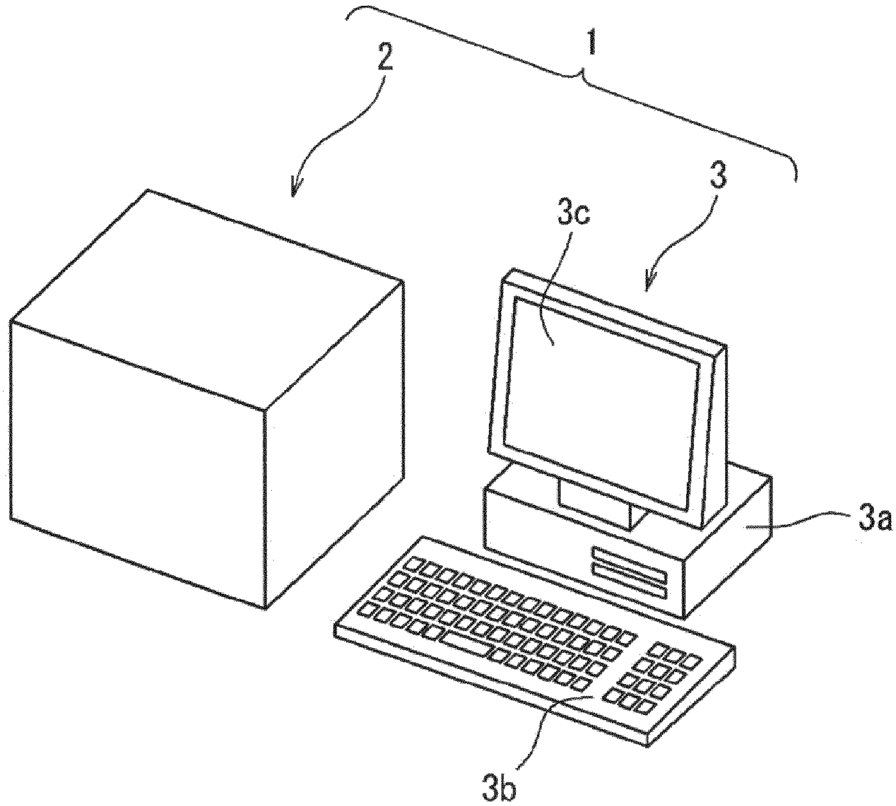

FIG. 9

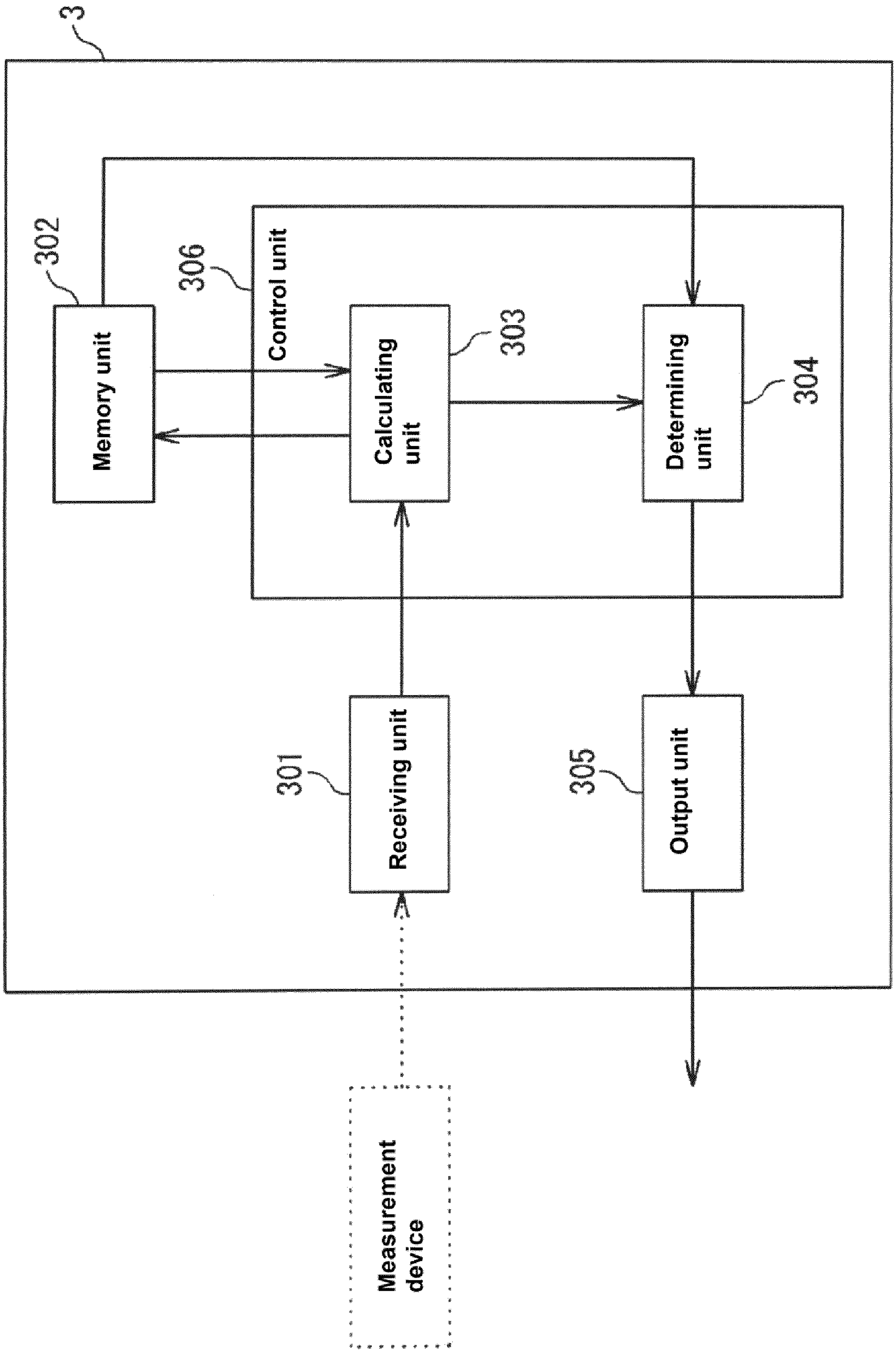
3
Memory unit
302
306
Control unit
Calculating unit
303
Determining unit
304
Receiving unit
301
Output unit
305
Measurement device

FIG. 10

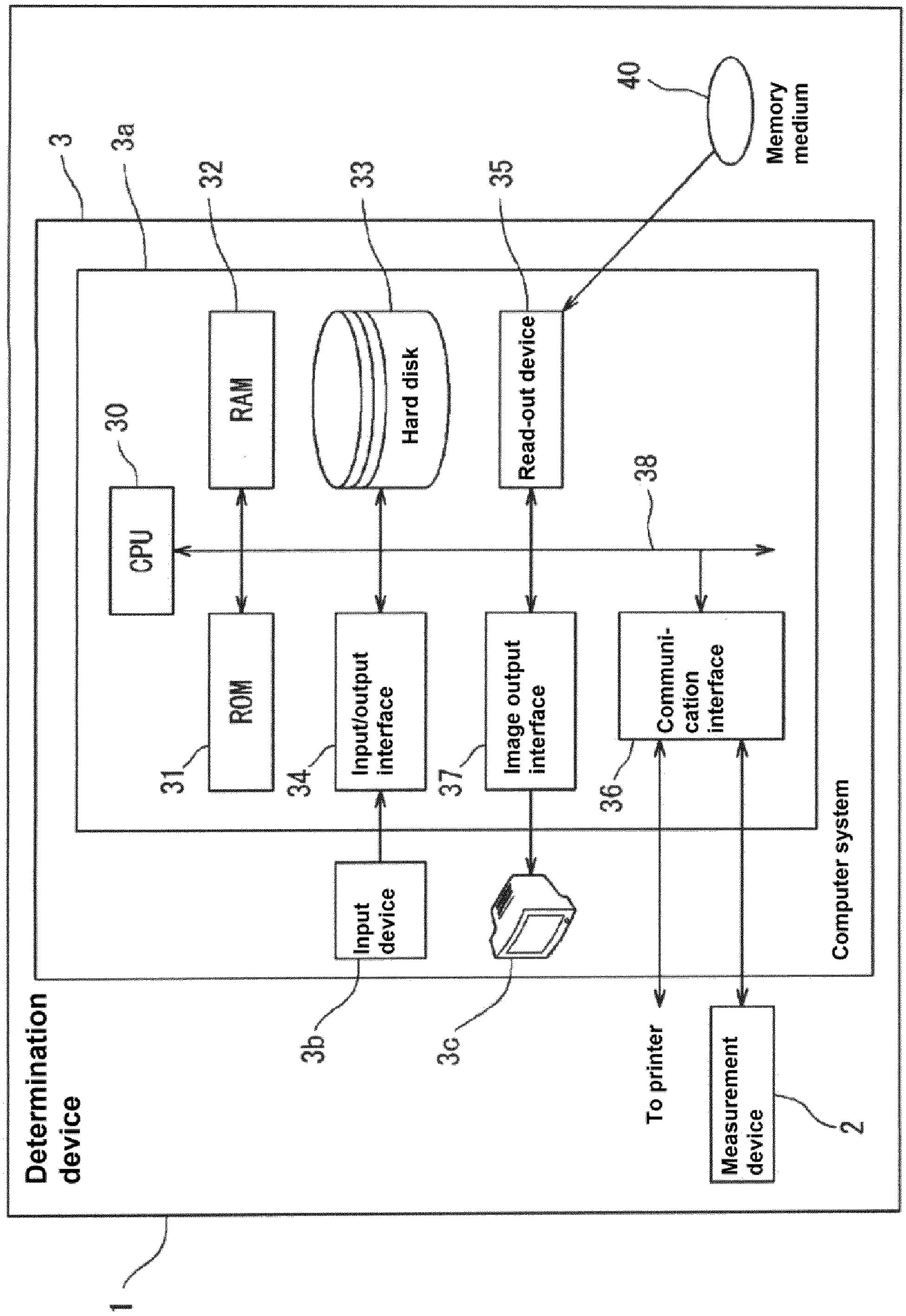
Determination device
1
3
3a
Computer system
30
CPU
31
ROM
32
RAM
33
Hard disk
34
Input/output interface
35
Read-out device
36
Communi-cation interface
37
Image output interface
38
40
Memory medium
3b
Input device
3c
To printer
Measurement device
2

FIG. 11

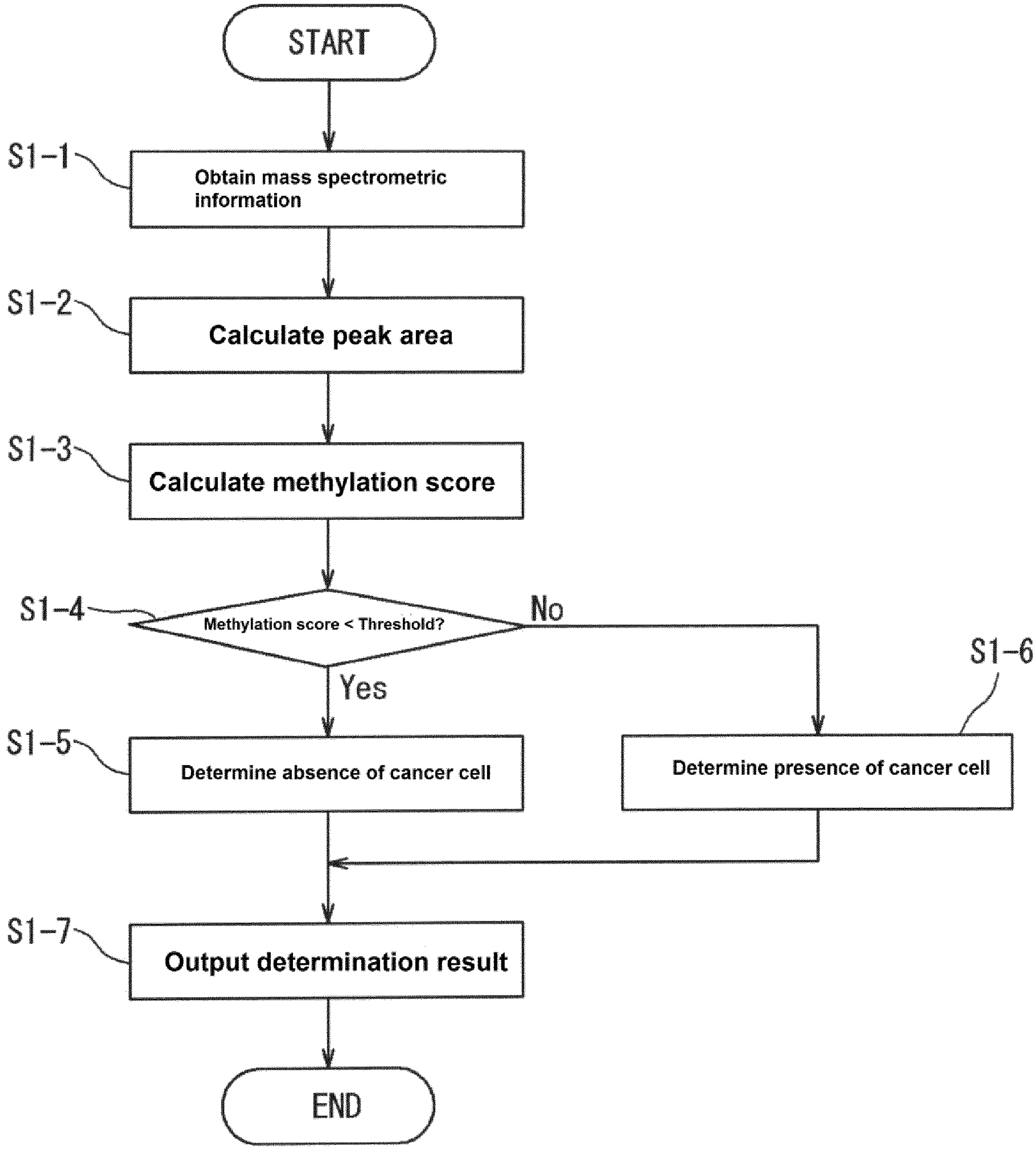
START
S1-1
Obtain mass spectrometric information
S1-2
Calculate peak area
S1-3
Calculate methylation score
S1-4
Methylation score < Threshold?
No
Yes
S1-5
Determine absence of cancer cell
S1-6
Determine presence of cancer cell
S1-7
Output determination result
END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2013113419 A
- JP 2014093759 A


### Non-patent literature cited in the description


- **LEE S. et al.** *Am J Pathol,* vol. 163 (4), 1371-78
- **SONG M-A et al.** *Plos One,* vol. 8 (2), e55761
- **NAZOR KL et al.** Recurrent variations in DNA methylation in human pluripotent stem cells and their differentiated derivatives. *Cell Stem Cell,* 2012, vol. 10 (5), 620-634
- **REINIUS LE et al.** Differential DNA Methylation in Purified Human Blood Cells: Implications for Cell Lineage and Studies on Disease Susceptibility. *PLoS One,* vol. 7 (7), e41361